# EUROPEAN PATENT SPECIFICATION

(11) **EP 4 356 135 B1**
(45) Date of publication and mention of the grant of the patent: **10.12.2025**
(21) Application number: 22735381.0
(22) Date of filing: 17.06.2022
(51) Int. Cl.: G01N 33/543, C07K 14/00

(54) **THERMOSTABLE AFFINITY POLYPEPTIDES**
THERMOSTABILE AFFINITÄTSPOLYPEPTIDE
POLYPEPTIDES D'AFFINITÉ THERMOSTABLES

(30) Priority: 17.06.2021 EP 21180048
(43) Date of publication of application: 24.04.2024
(73) Proprietor: Roche Diagnostics GmbH, 68305 Mannheim (DE); F. Hoffmann-La Roche AG, 4070 Basel (CH)
(72) Inventor: EISENHAUER, Romina, 82377 Penzberg (DE); ENGEL, Alfred, 82377 Penzberg (DE); JUCKNISCHKE, Ute, 82377 Penzberg (DE); SCHRAEML, Michael, 82377 Penzberg (DE); STEFFEN, Wojtek, 82377 Penzberg (DE)
(74) Representative: Altmann Stößel Dick Patentanwälte PartG mbB
(86) International application number: PCT/EP2022/066584
(87) International publication number: WO 2022/263644

(56) References cited:
- WO-A1-2016/187580
- WO-A1-2017/100584
- WO-A2-2008/106650
- UNKNOWN: "UniParc - UPI000C07F256", 29 October 2017 (2017-10-29), XP055855936, Retrieved from the Internet <URL:https://www.uniprot.org/uniparc/UPI000C07F256> [retrieved on 20211028]
- UNKNOWN: "UniParc - UPI000BA4EECA", 10 April 2019 (2019-04-10), XP055855951, Retrieved from the Internet <URL:https://www.uniprot.org/uniparc/UPI000BA4EECA> [retrieved on 20211028]
- UNKNOWN: "UniParc - UPI000D1115E6", 29 March 2018 (2018-03-29), XP055855961, Retrieved from the Internet <URL:https://www.uniprot.org/uniparc/UPI000D1115E6> [retrieved on 20211028]
- KUHLMANN U C ET AL: "Specificity in protein-protein interactions: the structural basis for dual recognition in endonuclease colicin-immunity protein complexes", JOURNAL OF MOLECULAR BIOLOGY, ACADEMIC PRESS, UNITED KINGDOM, vol. 301, no. 5, 1 September 2000 (2000-09-01), pages 1163 - 1178, XP004461689, ISSN: 0022-2836, DOI: 10.1006/JMBI.2000.3945

## Description

The present disclosure relates to a method of determining an analyte in a sample, said method comprising (a) contacting said sample with (i) a binding compound binding to said analyte, said binding compound comprising a binding agent and a first partner of an affinity pair (first affinity partner); and (ii) a second partner of the affinity pair (second affinity partner) coupled to a solid surface, to an indicator, and/or to a second binding agent; and (b) determining said analyte based on complexes formed in step (a); wherein one of said first affinity partner and said second affinity partner is a polypeptide comprising the amino acid sequence of SEQ ID NO:1 or a sequence at least 50% identical thereto, and wherein the other of said first affinity partner and said second affinity partner is a polypeptide comprising the amino acid sequence of SEQ ID NO:2 or a sequence at least 50% identical thereto. The present disclosure also relates to a polypeptide comprising an amino acid sequence as specified in SEQ ID NO:1 or a sequence at least 50% identical thereto, wherein the amino acid at the position corresponding to position 77 in SEQ ID NO:1 is not a histidine; and to a polypeptide comprising an amino acid sequence as specified in SEQ ID NO:2 or a sequence at least 50% identical thereto, wherein (i) the amino acid at the position corresponding to position 17 in SEQ ID NO:2 is not a cysteine, in an embodiment is alanine, serine, leucine, isoleucine, or glycine and/or (ii) said polypeptide further comprises at least one functional peptide; and to fusion polypeptides, polypeptide complexes, polynucleotides and kit related to the aforesaid. Hereinafter, the terminology "embodiment" relates to the disclosure and only forms part of the invention if it falls within the scope of the claims, which define the scope of protection.

In immunoassays, the affinity pair widely used, e.g. for tethering a capture compound to a solid surface, is the biotin-streptavidin affinity pair; however, in particular in recent years, biotin supplementation in nutrition has become quite fashionable, which may lead to an increased biotin content in medical samples and may cause interferences in diagnostic tests relying on said interaction. Another widely used affinity pair is digoxigenin and anti-digoxigenin (i.e. an antibody against digoxigenin). Also here, interference by digoxigenin binding molecules - so-called dig-binding proteins - present in samples have been observed. In accordance, diagnostic tests avoiding the use of the affinity pairs used so far are desirable.

The E group Colicins have been subdivided into nine types (ColE1 to ColE9) and these fall into one of three cytotoxic classes; membrane-depolarising agents such as ColE1, non-specific DNases such as Colicins E2, E7, E8 and E9, and RNases such as Colicins E3, E5 and E6 (reviewed in Cascales et al. (2007) Microbiol Mol Biol Rev 71(1):158). The Protein-Protein Interactions in Colicin E9 DNase-Immunity Protein Complexes were described by (Wallis et al. 1995 Biochemistry 34(42):13743); the mode of interaction is based on a diffusion-controlled association and femtomolar binding for the cognate complex. Garinot-Schneider et al. ((1996) J Mol Biol 260(5):731) performed random and site-directed mutagenesis on Colicin E9 and identified highly conserved active site residues responsible for DNase activity. In the full length Colicin sequence a single His575Ala mutation completely inactivated the Colicin 9 enzymatic activity. Kuhlmann et al. ((2000) J Mol Biol 301:1163) resolved the 1.7 Å x-ray structure and describe the structural basis of the highly specific interaction of the E.coli derived Colicin E9 to Immunity Protein 9.

Colicins are mostly described from Enterobacteriaceae, particularly E. coli. In order to robustly use Colicins and Immunity Proteins in biotechnology, Enterobacteriaceae-derived Colicin/Immunity Protein-Protein Interactions (PPI) are limited in their application due to their instability. Keeble et al. ((2006) Biochemistry 45(10):3243) showed melting points of the E.coli complexes ColE2/IM2, ColE7/IM7, ColE8/IM8, ColE9/IM9 generally below 45 °C. However, protein purification based on a Colicin-immunity affinity pair has been proposed, e.g. in WO 2017/100584 A1.

Further analytic methods based on affinity pairs were proposed e.g. in WO 2008/106650 A2; methods of labeling biological moelcules are described e.g. in WO 2016/187580 A1. Amino acid sequences of proteins similar to colicin or immunity proteins were also described for Clostridium spec., Bacillus circulans, and Brevibacillus brevis (UniParc entries UPI000C07F256, UPI000BA4EECA, and UPI000D1115E6, respectively).

The technical problem underlying the present invention may be seen in the provision of means and methods complying with the aforementioned needs, avoiding the problems identified as far as possible. The technical problem is solved by the embodiments characterized in the claims and described herein below.

In accordance, the present invention relates to a method of determining an analyte in a sample according to claim 1; further disclosure relates to a method of determining an analyte in a sample, said method comprising
(a) contacting said sample with (i) a binding compound binding to said analyte, said binding compound comprising a binding agent and a first partner of an affinity pair (first affinity partner); and (ii) a second partner of the affinity pair (second affinity partner) coupled to a solid surface, to an indicator, and/or to a second binding agent; and
(b) determining said analyte based on complexes formed in step (a);
wherein one of said first affinity partner and said second affinity partner is the polypeptide comprising the amino acid sequence of SEQ ID NO:1 or a sequence at least 85% identical thereto, and wherein the other of said first affinity partner and said second affinity partner is the polypeptide comprising the amino acid sequence of SEQ ID NO:2 or a sequence at least 85% identical thereto.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" in an embodiment refer to "comprising one or more", i.e. are equivalent to "comprising at least one". In accordance, expressions relating to one item of a plurality, unless otherwise indicated, in an embodiment relate to at least one such item, in a further embodiment a plurality thereof; thus, e.g. identifying "a cell" relates to identifying at least one cell, in an embodiment to identifying a multitude of cells.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein, in an embodiment, are in vitro methods. The method steps may, in principle, be performed in any arbitrary sequence deemed suitable by the skilled person, but in an embodiment are performed in the indicated sequence; also, one or more, in an embodiment all, of said steps may be assisted or performed by automated equipment. Moreover, the methods may comprise steps in addition to those explicitly mentioned above, in particular those specified herein below.

As used herein, the term "standard conditions", if not otherwise noted, relates to IUPAC standard ambient temperature and pressure (SATP) conditions, i.e. in an embodiment, a temperature of 25°C and an absolute pressure of 100 kPa; also in an embodiment, standard conditions include a pH of 7. Moreover, if not otherwise indicated, the term "about" relates to the indicated value with the commonly accepted technical precision in the relevant field, in an embodiment relates to the indicated value ± 20%, in a further embodiment ± 10%, in a further embodiment ± 5%. Further, the term "essentially" indicates that deviations having influence on the indicated result or use are absent, i.e. potential deviations do not cause the indicated result to deviate by more than ± 20%, in a further embodiment ± 10%, in a further embodiment ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. In an embodiment, a composition consisting essentially of a set of components will comprise less than 5% by weight, in a further embodiment less than 3% by weight, in a further embodiment less than 1% by weight, in a further embodiment less than 0.1% by weight of non-specified component(s).

The term "polypeptide", as used herein, refers to a molecule consisting of a multitude, typically at least 20, amino acids that are covalently linked to each other by peptide bonds. Molecules consisting of less than 20 amino acids covalently linked by peptide bonds may also be referred to as "peptides". In an embodiment, the polypeptide comprises of from 50 to 1000, in a further embodiment of from 55 to 750, in a further embodiment of from 60 to 500, in a further embodiment of from 65 to 400 amino acids. In an embodiment, the polypeptide is comprised in a fusion polypeptide and/or a polypeptide complex. In an embodiment, the polypeptide is a binding compound comprising at least a binding agent and a first affinity partner as specified herein below. In an embodiment, the polypeptide comprises at least one sequence of at least three, in an embodiment at least five, in a further embodiment at least ten contiguous amino acids not present in the naturally occurring polypeptide; in an embodiment, said sequence additionally present is a further amino acid sequence as specified herein below. Polypeptides as specified may be comprised in fusion polypeptides comprising further amino acid sequences; a further amino acid sequence in an embodiment is one or more repeat of the amino acid sequence of the polypeptide as specified and/or an amino acid sequence of a functional polypeptide, i.e. a polypeptide providing at least one additional, in an embodiment auxiliary, function. Repeats of the amino acid sequence of the polypeptide may be identical repeats or may be repeats having at least 50% sequence identity to said polypeptide; in an embodiment, said repeats are essentially identical to said polypeptide. Repeats may be direct repeats, i.e. the repeat being arranged directly adjacent to the amino acid sequence of the polypeptide, or may be indirect repeats, i.e. the sequence of the polypeptide and of the repeat being repeat being intervened by one or more further amino acid sequences, e.g. of functional polypeptides as specified herein above. In an embodiment the same applies to multiple repeats, wherein any two repeats may be connected directly or indirectly and may have an independently selected % identity value as specified above.

A functional polypeptide optionally present in a fusion polypeptide may be any polypeptide the presence of which is deemed desirable by the skilled person. In an embodiment, the functional polypeptide is selected from a tag peptide, a linker peptide, an indicator polypeptide, and a further binding agent. The term "tag peptide" is known to the skilled person to relate to a heterologous amino acid sequence comprised in a polypeptide providing auxiliary functionality, in particular as an affinity tag (e.g. His6-tag, strep-tag, or GST-tag), solubilization tag (e.g. thioredoxin or poly(NANP) (SEQ ID NO:17)), chromatography tag (e.g. His6-tag or FLAG-tag), epitope tag (e.g. Myc-tag, FLAG-tag, or HA-tag), fluorescence tag (e.g. GFP), modification tag (e.g. transglutaminase peptide or a protease recognition sequence), and/or targeting tag (e.g. mediators of secretion, mediators of blood-brain-barrier passage, or cell-penetrating peptides). These tags are all well known in the art. The term "linker peptide" is also known to the skilled person. In an embodiment, the linker peptide, which may also be referred to as "linker", is a sequence of from 2 to 25, in an embodiment 3 to 10 amino acids, in an embodiment small amino acids, in particular independently selected from glycine, serine, alanine, valine, and proline. In an embodiment, the linker peptide comprises the sequence GGG, in a further embodiment the sequence GGGS (SEQ ID NO:7), in a further embodiment the sequence (GGGS)5 (SEQ ID NO:8), in a further embodiment the sequence GGSSGGAGSGGG (SEQ **ID** NO:9). The term "indicator polypeptide", as used herein, relates to each and every polypeptide having the property of providing at least one detectable physical feature, chemical feature, and/or electrochemical feature as specified herein below to a fusion polypeptide; thus, in an embodiment, the indicator polypeptide is a fluorescence tag or an enzyme such as a peroxidase. The term "further binding agent" is understood by the skilled person in view of the description provided herein below. In an embodiment the further binding agent is not a repetition of the binding agent present in a binding compound, i.e. in an embodiment has an amino acid sequence with less than 50% identity to the sequence of the binding agent. In an embodiment, the further binding agent binds to the same structural feature, in an embodiment immunological feature, of the analyte, or binds to a different structural feature, in an embodiment immunological feature, of the analyte, or binds to a structural feature, in an embodiment immunological feature, of a second analyte, wherein the second analyte is non-identical to the analyte.

The expression "the amino acid at the position corresponding to position X is not amino acid Y" is used herein in the meaning understood by the skilled person. Thus, the term "position corresponding to position X" in the context of a polypeptide sequence, in an embodiment, relates to the position in a polypeptide which is determined to be the position corresponding position X by aligning the polypeptide of interest to e.g. SEQ ID NO:1 or 2, respectively. In accordance, an amino acid position corresponding to position X in an embodiment is the position corresponding to position X as determined from sequence context, but not necessarily by position number. Also, the expression "is not amino acid Y" relates to any amino acid not being amino acid Y; in an embodiment, said amino acid is an alpha-amino acid, in a further embodiment an L-amino acid, in a further embodiment an L-alpha amino acid. In particular in the context of a polypeptide produced or producible in a host cell, the amino acid is a proteinogenic amino acid. Thus, in an embodiment, the expression "amino acid X is not histidine" is equivalent to the expression "amino acid X is selected from the list consisting of alanine, valine, leucine, isoleucine, aspartic acid, glutamic acid, phenylalanine, glycine, lysine, methionine, asparagine, glutamine, proline, arginine, serine, threonine, tryptophan, tyrosine, and cysteine" and the expression "amino acid X is not cysteine" is equivalent to the expression "amino acid X is selected from the list consisting of alanine, valine , leucine, isoleucine, aspartic acid, glutamic acid, phenylalanine, glycine, histidine, lysine, methionine, asparagine, glutamine, proline, arginine, serine, threonine, tryptophan, and tyrosine".

The degree of identity (e.g. expressed as "%identity") between two biological sequences, in an embodiment DNA, RNA, or amino acid sequences, can be determined by algorithms well known in the art. In an embodiment, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, in an embodiment over the whole length of the polynucleotide or polypeptide, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are in an embodiment employed to determine their optimal alignment and, thus, the degree of identity. In an embodiment, the default values of 5.00 for gap weight and 0.30 for gap weight length are used. In the context of biological sequences referred to herein, the term "at least 50% identical" includes at least 70% identity, in an embodiment at least 80% identity, in a further embodiment at least 85% identity, in a further embodiment at least 90% identity, in a further embodiment at least 95% identity, in a further embodiment at least 98% identity, in a further embodiment at least 99% identity. Also, the term "essentially identical" indicates a %identity value of at least 80%, in an embodiment at least 90%, in a further embodiment at least 98%, in a further embodiment at least 99%. As will be understood, the terms at least 50% identity and essentially identical both include 100% identity. The aforesaid applies to the term "essentially complementary" mutatis mutandis.

The term "fragment" of a biological macromolecule, in an embodiment of a polynucleotide or polypeptide, is used herein in a wide sense relating to any sub-part, in an embodiment subdomain, of the respective biological macromolecule comprising the indicated sequence, structure and/or function. Thus, the term includes sub-parts generated by actual fragmentation of a biological macromolecule, but also sub-parts derived from the respective biological macromolecule in an abstract manner, e.g. in silico. Thus, as used herein, an Fc or Fab fragment, but also e.g. a single-chain antibody, a bispecific antibody, and a nanobody may be referred to as fragments of an antibody.

Unless specifically indicated otherwise herein, the compounds specified, in particular the polynucleotides and polypeptides, may be comprised in larger structures, e.g. may be covalently or non-covalently linked to further sequences, in particular as specified herein above and below, or further compounds, e.g. carrier molecules, retardants, and other excipients.

As indicated herein above, the method of determining an analyte is an in vitro method. In an embodiment, the method is a diagnostic method, i.e. a method providing diagnosis of a disease, or, in an embodiment, a method per se not allowing diagnosis of disease but contributing to establishing a diagnosis by the medical practitioner, in an embodiment in combination with further diagnostic methods and or symptoms. The method may, however, also be a non-diagnostic method for determining an analyte in a non-medical sample, in an embodiment a sample not derived from a subject, e.g. in environmental analytics, industrial production analytics, e.g. quality control, or the like.

As used herein, the term "determining an analyte" refers to determining at least one detectable feature of an analyte, wherein the analyte in an embodiment is comprised in an analyte/binding compound complex. The detectable feature may be any feature deemed detectable by the skilled person. In an embodiment, the detectable feature is selected from the list consisting of a structural feature, a physical feature, a chemical feature, and an electrochemical feature. Structural features are all detectable features conferred by the chemical structure of the analyte, in particular immunological features, i.e. the property of being recognized or recognizable by a binding compound as specified herein below. Physical features are all features detectable by physical means, e.g. optical features such as absorption, transmission, color, reflection, fluorescence, radiation, and the like. Chemical features are all features detectable by at least one chemical reaction, e.g. the activity of producing a chemical compound, in an embodiment when provided with a co-substrate and/or a catalyst; as referred to herein, a chemical property may be the activity of being a catalyst, e.g. an enzyme. Electrochemical features include all features based on a chemical reaction and detectable by electric means, e.g. by measuring current or voltage, optionally after applying an external voltage; electrochemical features include in particular ECL features. As the skilled person understands, determining a detectable feature of an analyte in an embodiment also includes detecting the absence of said feature; e.g. in a competitive assay, a specifying compound carrying a chemical group conferring at least one detectable feature comprised in the specifying compound, which is absent from the analyte, may be determined. In an embodiment, the detectable feature is an immunological feature. The term "immunological feature", as used herein, relates to a structural feature of the analyte facilitating detection of the analyte in a sample by a binding compound, in particular an antibody or a derivative thereof. In an embodiment, said immunological feature facilitates identification, in a further embodiment, quantification of the analyte by immunological means. Accordingly, typical immunological features are features facilitating differentiation of said analyte from other chemical compounds in a sample.

In an embodiment, determining an analyte is establishing whether an analyte is present or absent in the sample at a concentration above the detection limit of the method, i.e., in an embodiment, the determining is qualitative. Methods of determining a detection limit are known to the skilled person. In a further embodiment, determining is determining semi-quantitatively or quantitatively the amount or concentration of an analyte in a sample. For semi-quantitative determining, the amount may be assigned e.g. to two or more pre-defined categories, e.g. above or not above a reference value, or low, medium, or high. For quantitative determination, either the absolute or precise amount of the analyte will be determined or the relative amount of the analyte will be determined. The relative amount may be determined in a case were the precise amount of an analyte can or shall not be determined. In said case, it can be determined whether the amount in which the analyte is present is increased or diminished with respect to a reference sample comprising said analyte in a pre-determined amount. For quantitative determination, any parameter correlating with the amount or concentration of the analyte in the sample or any value derived therefrom by standard mathematical and/or evaluation operations, including in particular multiplication, division, reciprocal formation, scaling, normalization, standardization, error correction, background correction, or mean or median calculation, may be determined and/or output.

The specific steps performed for determining an analyte, in particular analyte/binding compound complexes, will depend on the specific assay format chosen. **In** an embodiment, the assay is a competitive immunoassay, i.e. an immunoassay wherein an analyte competes with a specifying compound, e.g., in an embodiment a labeled derivative of the analyte, in binding to a binding compound, said binding compound in an embodiment being bound to a solid surface, and wherein the amount of specifying compound bound to said binding compound is determined. In a further embodiment, the assay is a sandwich immunoassay, in particular a sandwich-ECL assay, a sandwich-ELISA, or a double-antigen sandwich assay ("DAGS"). As specified in more detail herein below, in such case the binding compound may be a capture compound and/or a detection compound. In a sandwich assay, the analyte is bound to a capture compound, said binding compound in an embodiment being bound to a solid surface, and the amount of capture compound/analyte complexes is determined by binding of a detection compound as specified herein below via the analyte to analyte/capture compound complexes. In a DAGS assay, in an embodiment, the analyte is at least bivalent, i.e. carries at least one detectable feature at least twice and the binding compound and the detection compound may comprise the same structural feature binding the analyte. Thus, in an embodiment, the binding agent of the capture compound and the binding agent of the detection compound are essentially identical, in an embodiment are identical, i.e. the capture compound and the detection compound may differ only in that the capture agent is adapted to be bound to a solid surface, while the detection compound comprises an indicator.

The term "analyte", as used herein, relates to a chemical molecule, in an embodiment, an organic molecule, which shall be determined in a sample. In an embodiment, the analyte has a molecular mass of at least 100 (corresponding to 100 atomic mass units, and to 100 Da; 1 Da corresponding to 1.66× 10⁻²⁷ kg), in a further embodiment, at least 250, in a further embodiment, at least 500, or, in a further embodiment, at least 1000. In an embodiment, the analyte has a molecular mass of at most 2500, in an embodiment at most 1000. In an embodiment, the analyte is a biological molecule, in a further embodiment, the analyte is a biological macromolecule. In a further embodiment, the analyte is a polypeptide. In an embodiment, the analyte binds to the binding compound with sufficient affinity to allow detection of an analyte/binding compound complex. In an embodiment, the analyte is Troponin T and/or IGF-1 (Insulin-like growth factor-1). In an embodiment, the analyte is a polypeptide antigen produced by an infectious agent, e.g., by a virus or bacterium, or an antibody, e.g., an antibody produced by a subject against an antigen produced by an infectious agent. In an embodiment, the analyte is a polypeptide, in a further embodiment, is an antibody against a viral antigen, in a further embodiment, against a viral polypeptide, or, in a further embodiment, against a viral capsid polypeptide. In an embodiment, the viral capsid polypeptide is a Hepatitis virus capsid polypeptide, in an embodiment, a Hepatitis B virus (HB) capsid polypeptide, or, in a further embodiment, a HB core (HBc) antigen, or in a further embodiment, a SARS-CoV-2 antigen, in particular SARS-CoV-2 spike or nucleocapsid antigen. In an embodiment, in particular in case the method uses a competitive assay format, analyte is Anti-HBc, Anti-HAV (anti-Hepatitis A virus), Anti-HBe (anti-Hepatitis B e-antigen), Folate, Folate RBC (red blood cell), Anti-TSH-R, Vitamin D total, Vitamin B12, Tyroxin T4, FT 4 (free thyroxine), Tyroxin T3, FT 3 (free triiodothyronine), Testosteron, Progesterone, Digitoxin, Anti-TG, Anti-TPO (anti-Thyroid peroxidase), DGEA, or Estradiol. In an embodiment, in particular in case the method uses a DAGS assay format, the analyte may in particular be an antibody, in particular Anti-Toxoplasma IgG, Anti-rubella IgG, Anti-HBs 1G, HCV (hepatitis C virus), CMV (cytomegalovirus) IgG, or an antibody diagnostic for e.g. HCV infection, Syphilis, HTLV (Human T-cell lymphotropic virus) infection, or Chagas (American trypanosomiasis) infection, or SARS-CoV-2 infection

The term "sample", as used herein, relates to a sample known or suspected to comprise the analyte. In an embodiment, the sample is or comprises a sample of a body fluid, a sample from a tissue or an organ, or a sample of wash/rinse fluid or a swab or smear obtained from an outer or inner body surface. Body fluid samples include samples of blood, plasma, serum, urine, saliva, and lacrimal fluid. Samples can be obtained by use of brushes, (cotton) swabs, spatula, rinse/wash fluids, punch biopsy devices, puncture of cavities with needles or lancets, or by surgical instrumentation. However, samples obtained by well-known techniques including, in an embodiment, scrapes, swabs or biopsies from the urogenital tract, perianal regions, anal canal, the oral cavity, the upper aerodigestive tract and the epidermis are also included as samples. Cell-free fluids may be obtained from the body fluids or the tissues or organs by lysing techniques such as homogenization and/or by separating techniques such as filtration or centrifugation. It is to be understood that the sample may be further processed in order to carry out the method. Particularly, cells may be removed from the sample by methods and means known in the art, calcium ions may be complexed to prevent coagulation, coagulation may be induced, and the like. Moreover, at least one analyte may be enriched, extracted, and/or purified from the sample by methods and means known in the art. Thus, the term sample also may relate to preparations comprising or suspected to comprise at least one analyte which are diluted, enriched, purified and/or extracted from a sample. In an embodiment, the sample is a sample not derived from a subject, e.g. a water sample, a sewage sample, a food sample, or the like.

As the skilled person will appreciate, the term "specific binding" and grammatical variations thereof are used herein to indicate that other compounds, typically biomolecules, present in a sample do not significantly bind to a ligand, in particular a binding compound. In an embodiment, the dissociation constant of the analyte/binding compound complex is at least a factor of 5, in an embodiment at least a factor of ten, in a further embodiment at least a factor of 100, lower than the dissociation constant of a complex between any non-analyte compound in the sample and the binding compound.

As used herein, the term "binding compound" relates to a chemical molecule binding to the analyte as specified herein above; the binding between the analyte and the binding compound in an embodiment is direct, i.e. in an embodiment there is a direct molecular interaction between the binding compound and the analyte. The binding between the analyte and the binding compound may, however, also be indirect. In an embodiment, the binding between the analyte and the binding agent is specific; in an embodiment, the binding is group specific for a class of compounds sharing a structural feature, e.g. specificity for IgG molecules; more preferable, said binding is analyte specific, i.e., in an embodiment, the binding between the analyte and the binding compound is a specific binding as specified herein above. In an embodiment, the dissociation constant (K_{d}) of the analyte/binding compound complex is at most 10⁻⁸ mol/l, in a further embodiment at most 10⁻⁹ mol/l, in a further embodiment at most 10⁻¹⁰ mol/l, in a further embodiment at most 10⁻¹¹ mol/l, in a further embodiment, at most 10⁻¹² mol/l, in an embodiment under standard conditions, in a further embodiment under the conditions specified herein in the Examples.

As referred to herein, the binding compound comprises a binding agent and a first partner of an affinity pair (first affinity partner), both as specified herein. The binding agent and the first affinity partner, in an embodiment, are covalently connected. In a further embodiment at least a part of the binding agent and the first binding partner are comprised in a fusion polypeptide; e.g. in case the binding agent is an antibody or fragment thereof, the heavy chain or a fragment derived therefrom may be comprised in said fusion polypeptide, and/or the light chain or a fragment derived therefrom may be comprised in said fusion polypeptide. In an embodiment, the binding agent and the first binding partner in the binding compound are intervened by a linker peptide, in an embodiment comprising the sequence GGSSGGAGSGGG (SEQ **ID** NO:9). In an embodiment, the binding compound comprises, in an embodiment consists of, the amino acid sequence of SEQ ID NO:10, in an embodiment covalently connected via at least one disulfide bridge to a polypeptide comprising, in an embodiment consisting of, SEQ ID NO:11. In an embodiment, the binding compound comprises, in an embodiment consists of, the amino acid sequence of SEQ ID NO:12, in an embodiment covalently connected via at least one disulfide bridge to a polypeptide comprising, in an embodiment consisting, of SEQ ID NO:11.

The term "binding agent", as used herein, refers to any and all compounds binding, in an embodiment specifically binding, to an analyte. Binding agents are, in principle, known to the skilled person. In an embodiment, the binding agent is an organic molecule, in a further embodiment, a biological macromolecule, in particular a polypeptide as specified herein above. In an embodiment, the binding agent comprises an antibody, an aptamer, an Anticalin, a Designed Ankyrin Repeat Protein, a receptor, or a fragment of one of the aforesaid having the activity of binding to the analyte, in an embodiment comprises an antibody or fragment thereof having the activity of binding to the analyte. In an embodiment, the binding agent binds indirectly to the analyte of the present invention with sufficient affinity to allow detection of the complex comprising analyte and binding compound; i.e., in such case, the binding agent is an indirect ligand. The term "indirect binding", as used herein, relates to a binding wherein the ligand does not directly contact the analyte, but contacts a chemical molecule binding the analyte, in an embodiment specifically binding the analyte, wherein, in an embodiment, said molecule binding the analyte is a molecule directly binding the analyte, i.e. is a direct ligand. In an embodiment, the binding agent is a direct ligand of the analyte.

The term "antibody" is known to the skilled person. As used herein, the term includes monoclonal antibodies, multispecific antibodies (e.g. bispecific antibodies) formed from at least two non-identical antibodies, and antibody fragments as long as they exhibit the desired binding activity as specified elsewhere herein. In an embodiment, the antibody is a full-length antibody or an antibody fragment. The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region. "Antibody fragments" comprise a portion of an intact antibody, in an embodiment, comprising the antigen-binding region thereof. Examples of antibody fragments include Fab, Fab', F(ab')2, and Fv fragments; diabodies; linear antibodies; single-chain antibody molecules, nanobodies; and multispecific antibodies formed from antibody fragments.

As used herein, the term "aptamer" relates to a macromolecule specifically binding its interaction partner. Aptamers can be peptide or polynucleotide aptamers and are in principle known to the skilled person. In an embodiment, the aptamer is a peptide aptamer, i.e. a polypeptide specifically binding its interaction partner and comprising 8-80 amino acids, in an embodiment 10-50 amino acids, in a further embodiment 15-30 amino acids. Aptamers can e.g. be isolated from randomized libraries, e.g. randomized peptide expression libraries, in a suitable host system like baker's yeast. As used herein, the term "anticalin" relates to an artificial polypeptide derived from a lipocalin specifically binding its interaction partner. Similarly, a "Designed Ankyrin Repeat Protein" or "DARPin", as used herein, is an artificial polypeptide comprising several ankyrin repeat motifs and specifically binding its interaction partner. The term "receptor", as used herein, includes each and every biological macromolecule, in an embodiment polypeptide, having the biological function of detecting the presence of a chemical compound in a biological system, e.g. a cell. The receptor may be the receptor a produced by a cell or a fragment thereof having the activity of binding the analyte. Appropriate receptors are known in the art, e.g. the estrogen receptor, folate receptor, vitamin D binding protein, ACE-2 (angiotensin-converting enzyme).

The term "affinity pair" is understood by the skilled person. In an embodiment, the term relates to a pair of compounds, in an embodiment polypeptides, forming a complex in an aqueous solution, in an embodiment under standard conditions. In an embodiment, the complex between the components of the affinity pair has a dissociation constant of at most 10⁻⁹ mol/l, in an embodiment at most 10⁻¹⁰ mol/l, in a further embodiment at most 10⁻¹¹ mol/l, in a further embodiment at most 10⁻¹² mol/l, in a further embodiment at most 10⁻¹³ mol/l, in a further embodiment at most 10⁻¹⁴ mol/l. In an embodiment, the affinity pair comprises, in a further embodiment consists of, a first affinity partner and a second affinity partner as specified herein. As referred to herein, the affinity pair comprises one affinity partner comprising the amino acid sequence of at least a fragment of the Colicin polypeptide from Geobacillus thermoglucosidasius (also referred to as Parageobacillus thermoglucosidasius) or a sequence at least 50% identical thereto, and another affinity partner comprising at least a fragment of the corresponding immunity polypeptide from the same organism or a sequence at least 50% identical thereto.

The amino acid sequence of the G. thermoglucosidasius Colicin polypeptide is available from public databases, e.g. Genbank Acc No: OUM93572.1 or UniProtKB Acc No: A0A1Y3Q1S7 and has the activity of binding the corresponding immunity polypeptide with an affinity as specified herein. In accordance, a fragment of the Colicin polypeptide in an embodiment is a fragment having said activity. In an embodiment, the one affinity partner comprises the amino acid sequence of SEQ ID NO:1 or a sequence at least 85% identical thereto, in a further embodiment at least 90% identical thereto, in a further embodiment at least 95% identical thereto, in a further embodiment at least 98% identical thereto, in a further embodiment at least 99% identical thereto. Thus, in the method of determining an analyte, the one affinity partner may comprise the wildtype sequence of the G. thermoglucosidasius Colicin polypeptide or a fragment and/or mutein thereof. In an embodiment, in the amino acid sequence of the one affinity partner, the amino acid at the position corresponding to position 77 in SEQ ID NO:1 is not a histidine; in an embodiment is alanine, glycine, leucine, or isoleucine, in a further embodiment is alanine. Also in an embodiment, in the amino acid sequence of the one affinity partner, the amino acid at the position corresponding to position 35 in SEQ ID NO:1 is not a cysteine, in an embodiment is alanine, serine, leucine, isoleucine, or glycine, in a further embodiment is alanine. Thus, in an embodiment, the one affinity partner comprises, in a further embodiment consists of, the amino acid sequence of SEQ ID NO:3. In a further embodiment, the one affinity partner further comprises at least one functional peptide as specified herein above, in a further embodiment at least one linker peptide, an affinity tag, and/or a modification tag. In an embodiment, the one affinity partner further comprises a (His)6 or (His)8 tag, at least one GGG- or a GGS-linker, and a transglutaminase peptide. In a further embodiment the one affinity partner further comprises a glycine residue preceding the sequence of SEQ ID NO:3. In an embodiment, the one affinity partner comprises, in an embodiment consists of, the amino acid sequence of SEQ ID NO:13.

The amino acid sequence of the G. thermoglucosidasius immunity protein is available from public databases, e.g. Genbank Acc No: OAO88314.1 or UniProtKB Acc No: A0A178U4V9 and has the activity of binding the corresponding Colicin polypeptide with an affinity as specified herein. In accordance, a fragment of the immunity polypeptide in an embodiment is a fragment having said activity. In an embodiment, the other affinity partner comprises the amino acid sequence of SEQ ID NO:2 or a sequence at least 85% identical thereto, in a further embodiment at least 90% identical thereto, in a further embodiment at least 95% identical thereto, in a further embodiment at least 98% identical thereto, in a further embodiment at least 99% identical thereto. Thus, in the method of determining an analyte, the one affinity partner may comprise the wildtype sequence of the G. thermoglucosidasius immunity polypeptide. In an embodiment, in the amino acid sequence of the other affinity partner, the amino acid at the position corresponding to position 17 in SEQ ID NO:2 is not a cysteine, in an embodiment is alanine, serine, leucine, isoleucine, or glycine, in a further embodiment is alanine. Thus, in an embodiment, the other affinity partner comprises, in a further embodiment consists of, the amino acid sequence of SEQ ID NO:4. In a further embodiment, the other affinity partner further comprises at least one functional peptide as specified herein above, in a further embodiment at least one linker peptide, an affinity tag, and/or a modification tag. In an embodiment, the one affinity partner further comprises a (His)6 or (His)8 tag, at least one GGG- or a GGS-linker, and a transglutaminase peptide. In a further embodiment the other affinity partner further comprises a glycine residue preceding the sequence of SEQ ID NO:4. In an embodiment, the one affinity partner comprises, in an embodiment consists of, the amino acid sequence of SEQ ID NO:14.

The terms "first partner of an affinity pair" and "first affinity partner", "second partner of an affinity pair" and "second affinity partner", "one affinity partner" and "other affinity partner", are used herein solely to differentiate between the two partners of the affinity pair; thus, the expression "first partner" does not relate to any timely or spatial relation to other compounds, but is used only for the purpose of differentiation from the "second partner" of an affinity pair. The first affinity partner and the second affinity partner, and the one and the other affinity partner, together constitute the affinity pair as specified herein above, respectively. As indicated herein above, the first affinity partner may be the polypeptide comprising the amino acid sequence of SEQ ID NO:1 or a sequence at least 85% identical thereto, or may be a polypeptide comprising the amino acid sequence of SEQ ID NO:2 or a sequence at least 85% identical thereto. Also, the second affinity partner may be the polypeptide comprising the amino acid sequence of SEQ ID NO:1 or a sequence at least 85% identical thereto, or may be a polypeptide comprising the amino acid sequence of SEQ ID NO:2 or a sequence at least 85% identical thereto. In view of the above, it is understood by the skilled person that in case the first affinity partner is the polypeptide comprising the amino acid sequence of SEQ ID NO:1 or a sequence at least 85% identical thereto, the second affinity partner is the polypeptide comprising the amino acid sequence of SEQ ID NO:2 or a sequence at least 85% identical thereto, and vice versa. In accordance, in the method of determining an analyte both partners of the affinity pair are used. In an embodiment, the first affinity partner is a polypeptide comprising the amino acid sequence of SEQ ID NO:1 or a sequence at least 85% identical thereto, and the second affinity partner is a polypeptide comprising the amino acid sequence of SEQ ID NO:2 or a sequence at least 85% identical thereto.

In an embodiment, the binding compound is a capture compound. As used herein, the term "capture compound" relates to a binding compound as specified herein above bound to a solid surface or adapted to be bound to a solid surface.

In a further embodiment, the binding compound is a detection compound. The term "detection compound", as used herein, relates to a binding compound as specified herein above bonded to an indicator. In an embodiment, the detection compound is not bound to a solid surface and not adapted to be bound to a solid surface.

The term "indicator", as used herein, relates to a compound adapted for making the presence of a molecule or complex comprising said indicator detectable. Typically, the indicator has a detectable property, typically an optical or/and enzymatic property.

The term "optical property", as used herein, relates to any property which can be detected by an optical instrument. Specifically, the optically determinable property may be or may comprise at least one property selected from the group consisting of: a reflection property, a transmission property, an emission property, a scattering property, a fluorescence property, a phosphorescence property, a diffraction property, and a polarization property. Further optical properties envisaged by the present invention are color, fluorescence, luminescence, or refraction. In an embodiment, an optically determinable property as referred to herein refers to a property of a chemical compound which can be optically detected such as light absorption, light emission, light remission, or properties associated therewith. It will be understood that detecting an optically determinable property as used herein encompasses the detection of the presence of a property which was not detectable before, the detection of the absence of a property which has been detected before, and the detection of quantitative changes of a property, i.e., the detection of the change of the signal strength which correlates to the extent of the change of the at least one optical property. It is understood that the term "optically determinable property", in an embodiment, also relates to electrochemiluminescence, which is also known as electrogenerated chemiluminescence.

The term "enzymatic property", as used herein, relates to a property of an indicator of producing a detectable product from a substrate by means of biological catalysis. Accordingly, an enzymatic property is typically conferred by the presence of a polypeptide having said enzymatic property in said indicator. Typically, the enzymatic property is at least one enzymatic activity selected from the group consisting of: phosphatase activity (e.g. in alkaline phosphatase), peroxidase activity (e.g. in horseradish peroxidase), and glycosidase activity (e.g. in beta-galactosidase). Typical substrates for enzymatic activities are well-known in the art. Typically, said enzymatic activity produces a product having a determinable optical property as specified herein above, or/and said enzymatic activity produces a product being determinable by an electrical instrument.

As used herein, the term "solid surface" relates to any suitable solid surface adapted for binding a second binding partner and adapted for being separated, e.g., by physical means, from a sample. In an embodiment, said solid surface is a surface of a bead, in an embodiment, a microbead, e.g. a magnetic or paramagnetic microbead. In an embodiment, said surface is adapted to improve binding of the second binding partner, e.g. by attaching, covalently or non-covalently, molecules binding a substructure of the second binding partner. Typical molecules binding a substructure of the second binding partner are, e.g. antibodies, streptavidin, complexed Nickel ions, and the like. In a further embodiment, the solid surface binds said second binding partner by covalent or non-covalent bonds, e.g. by hydrophobic interaction; thus, a specific adaptation of a polypeptide for binding to a solid surface may not be required. Thus, in an embodiment, said solid surface is a surface of a multi-cluster plate. In an embodiment, the surface of the multi-cluster plate is pretreated to increase affinity and/or capacity for binding of a capture compound. Suitable pretreatments are known in the art. In an embodiment, the second binding partner binds directly to the solid surface. The second binding partner may, however, also be bound indirectly to the solid surface, e.g. an antibody specifically recognizing the second binding partner may be bound to the solid surface, an antibody specifically recognizing a structural feature of a fusion polypeptide comprising the second binding partner may be bound to the solid surface. In the latter case, said structural feature of a fusion polypeptide comprising the second binding partner may, e.g. be a binding agent, in particular an antibody or fragment thereof.

Step (a) of the method of determining an analyte comprises contacting said sample with (i) a first affinity partner; and (ii) a second affinity partner. The term "contacting" as used in the context of the methods specified is understood by the skilled person. In an embodiment, the term relates to bringing at least one compound in physical contact with a sample and/or with a further compound and thereby, e.g. allowing the sample and the compound to interact. In particular, the term relates to bringing a first affinity partner and a second affinity partner in physical contact with a sample. Step (a) may comprise bringing the aforesaid compounds into contact with further compounds and elements, depending on the assay format chosen; e.g. in assays relying on removal of non-complexed sample constituents via binding of complexes to a solid surface, the admixture may be further contacted with a solid surface. In an embodiment, in case the binding compound is a capture compound, said contacting may be performed before step (a), e.g. by binding the second affinity partner to a solid surface before sample addition, may be performed during step (a), e.g. by admixing beads as specified herein above to the admixture, or may be performed after step (a), e.g. by binding pre-formed complexes to a solid surface. In an embodiment, in case the binding compound is a detection compound, said contacting may be performed before step (a), e.g. admixing a detection compound before sample addition, may be performed during step (a), e.g. by admixing a detection compound to the admixture, e.g. in case the binding compound is a detection compound, or after step (a), e.g. by binding pre-formed binding compound/analyte complexes to a detection compound.

The method of determining an analyte comprises step (b) determining the analyte based on complexes formed in step (a). The term "complex" is understood by the skilled person to relate to the complex formed in step (a) relevant for determining the analyte. Thus, in an embodiment the complex is a complex comprising at least the analyte and the binding agent. The complex may form at any time during the method before step (b), and may form sequentially or in one step. Sequential complex formation may be induced by providing a capture compound bound to a solid surface, admixing a sample, optionally washing away non-bound components of the sample, and adding a detection compound, in an embodiment followed by a further washing step. One-step complex formation may be induced e.g. by admixing a capture compound which is optionally bound to a solid surface, e.g. a bead, a sample, and a detection compound, optionally followed by washing away all compounds which are not bound directly or indirectly to said solid surface, wherein at least one of the capture compound and the detection compound is a binding compound as specified herein. For determining the analyte, in an embodiment the amount of the aforesaid complex formed in step (a) is determined. In an embodiment, liquid phase and solid phase components are separated from each other prior to determining the analyte in any of the liquid or solid phase or both. In an embodiment, the amount is determined indirectly e.g. by determining the amount of complexes comprising the binding agent, in an embodiment as a capture agent, and a specifying compound. In a further embodiment, the amount is determined directly by determining the amount of complexes comprising the binding agent and the analyte; in an embodiment by determining the amount of indicator comprised in complexes comprising a capture compounds, the analyte, and a detection compound. Thus, in case the binding compound is a capture compound, in an embodiment a detection compound is added before, during, or after step (a); and in case the binding compound is a detection compound, a capture compound in an embodiment is added. In view of the above, the skilled person understands that in case the binding compound is a capture compound, it is immaterial when the binding of the second binding partner to a solid surface occurs provided this occurs before the measurement in step (b), i.e. provided the second affinity partner is bound to a solid surface in step (b).

The term "specifying compound", for which also the term "specifier" may be used, is in principle known to the skilled person and relates to a compound competing with an analyte for binding to the binding compound, bonded to an indicator. Typically, the specifying compound is structurally similar to the analyte. Typically, the specifying compound comprises the substructure of the analyte bound by the capture compounds, bonded to an indicator. In an embodiment, the specifying compound is a compound comprising the analyte and an indicator as structural elements, or the specifying compound consists of the analyte covalently bonded to an indicator.

As used herein, an expression indicating that two compounds "compete in binding" to a binding compound relates to the property of molecules of said compounds being unable to bind to essentially the same binding site of said binding compound at the same time. Thus, typically, in case an analyte and a specifying compound compete in binding to a binding compound and in case said binding compound has one binding site for said analyte or specifying compound, at each point in time, only one molecule of said analyte or specifying compound can be bound to said binding compound. It will be understood by the skilled person that the above applies mutatis mutandis in case the binding compound has two or more binding sites for the analyte or specifying compound. The skilled person knows how to determine competition in binding. In an embodiment, competing in binding to a binding compound is binding with the same or essentially the same substructure of said analyte or specifying compound. In a further embodiment, in case the analyte is a polypeptide, the epitope bound by the binding compound is also present in the specifying compound.

In an embodiment, the affinity pair of the invention is used to couple two binding agents specifically binding to two non-identical analytes, e.g. to provide a capture compound binding two non-identical analytes to a solid surface. Thus, the present invention also relates to a method of determining at least two analytes in a sample, said method comprising
(a) contacting said sample with (i) a first binding compound binding to a first analyte, said first binding compound comprising a first binding agent and a first partner of an affinity pair (first affinity partner); (ii) a second binding compound binding to a second analyte, said second binding compound comprising a second binding agent and a second partner of said affinity pair (second affinity partner); and (iii) a capture agent coupled to a solid surface, wherein said capture agent binds to at least one of said first binding compound and said second binding compound; and
(b) determining said analyte based on the complexes formed in step (a);
wherein one of said first affinity partner and said second affinity partner is a polypeptide comprising the amino acid sequence of SEQ ID NO:1 or a sequence at least 50% identical thereto, and wherein the other of said first affinity partner and said second affinity partner is a polypeptide comprising the amino acid sequence of SEQ ID NO:2 or a sequence at least 50% identical thereto.

The term "capture agent", as used herein, relates to each and every compound binding to the first binding agent, the second binding agent, the first affinity partner, and/or the second affinity partner, and binding to a solid surface. The capture agent, in an embodiment, is a binding agent specifically binding at least one of the aforesaid structures.

Advantageously, it was found in the work underlying the present invention that the Colicin and immunity polypeptides from Geobacillus thermoglucosidasius form complexes which are highly stable and therefore particularly suitable for application as an affinity pair in immunoassays, e.g. to bind a complex of interest to a solid surface. Moreover, it was surprisingly found that the G. thermoglucosidasius Colicin and immunity polypeptides have a high biophysical stability and therefore are suitable for use in analytical assays.

The definitions made above apply mutatis mutandis to the following. Additional definitions and explanations made further below also apply for all embodiments described in this specification mutatis mutandis.

The present invention further relates to a polypeptide according to claim 9; further disclosure relates to a polypeptide comprising an amino acid sequence as specified in SEQ ID NO:1 or a sequence at least 50% identical thereto, wherein the amino acid at the position corresponding to position 77 in SEQ ID NO:1 is not a histidine, in an embodiment is alanine, glycine, leucine, or isoleucine.

In accordance, the aforesaid polypeptide is a mutein of the G. thermoglucosidasius Colicin as specified herein above and is also referred to as a "Colicin polypeptide". As referred to herein, said designation as Colicin polypeptide implies the activity of binding to a cognate immunity polypeptide, but not necessarily the nuclease activity of the Colicin. Thus, the Colicin polypeptide in an embodiment is devoid of detectable DNase activity. Moreover, the Colicin polypeptide is thermostable for at least 5 min at a temperature of 60°C, in an embodiment has a melting point of at least 60°C. Methods of determining thermostability are provided herein in the Examples. In an embodiment, in the Colicin polypeptide the amino acid at the position corresponding to position 35 in SEQ ID NO:1 is not a cysteine, in an embodiment is alanine, serine, leucine, isoleucine, or glycine. In a further embodiment, said Colicin polypeptide further comprises at least one functional peptide as specified herein above, in a further embodiment at least one linker peptide, an affinity tag, and/or a modification tag. In an embodiment, the Colicin polypeptide further comprises a G residue immediately preceding said amino acid sequence, in an embodiment further comprises the amino acid sequence MG immediately preceding said amino acid sequence, wherein, in an embodiment, said amino acid sequence MG is the N-terminal sequence of said Colicin polypeptide. In an embodiment, the Colicin polypeptide further comprises a transglutaminase peptide, in an embodiment comprising the amino acid sequence YRYRQ (SEQ ID NO:15); a tag peptide, in an embodiment comprising the amino acid sequence (His)6 (SEQ ID NO:5), in a further embodiment (His)8 (SEQ ID NO:6), and/or at least one linker peptide, in an embodiment as specified herein above. In an embodiment, the Colicin polypeptide comprises the sequence of SEQ ID NO:3, in a further embodiment of SEQ ID NO:16, in a further embodiment of SEQ ID NO:13. In an embodiment, the Colicin polypeptide comprises one of the aforesaid amino acid sequences of this paragraph or a sequence at least 85%, in a further embodiment at least 90%, in a further embodiment at least 95%, in a further embodiment at least 98%, identical thereto.

In an embodiment, the Colicin polypeptide is comprised in a fusion polypeptide, said fusion polypeptide in an embodiment further comprising a binding agent.

The present invention also relates to a polypeptide according to claim 10; further disclosure relates to a polypeptide comprising an amino acid sequence as specified in SEQ ID NO:2 or a sequence at least 50% identical thereto, wherein (i) the amino acid at the position corresponding to position 17 in SEQ ID NO:2 is not a cysteine, in an embodiment is alanine, serine, leucine, isoleucine, or glycine and/or (ii) said polypeptide further comprises at least one functional peptide.

In accordance, the aforesaid polypeptide is a mutein of the G. thermoglucosidasius immunity polypeptide as specified herein above and is also referred to as an "immunity polypeptide". As referred to herein, said designation as immunity polypeptide implies the activity of binding to a cognate Colicin polypeptide. Moreover, the immunity polypeptide is thermostable for at least 5 min at a temperature of 60°C, in a further embodiment has a melting point of at least 60°C. In an embodiment, in the immunity polypeptide the amino acid at the position corresponding to position 17 in SEQ ID NO:2 is not a cysteine, in an embodiment is alanine, serine, leucine, isoleucine, or glycine. In a further embodiment, said immunity polypeptide further comprises at least one functional peptide as specified herein above, in a further embodiment at least one linker peptide, an affinity tag, and/or a modification tag. In an embodiment, the immunity polypeptide further comprises a G residue immediately preceding said amino acid sequence, in an embodiment further comprises the amino acid sequence MG immediately preceding said amino acid sequence, wherein, in an embodiment, said amino acid sequence MG is the N-terminal sequence of said immunity polypeptide. In an embodiment, the immunity polypeptide further comprises a transglutaminase peptide, in an embodiment comprising the amino acid sequence YRYRQ (SEQ ID NO: 15); a tag peptide, in an embodiment comprising the amino acid sequence (HIS)6 (SEQ ID NO:5), in a further embodiment (His)8 (SEQ ID NO:6), and/or at least one linker peptide, in an embodiment as specified herein above. In an embodiment, the immunity polypeptide comprises the sequence of SEQ ID NO:4, in a further embodiment of SEQ ID NO:14. In an embodiment, the immunity polypeptide comprises one of the aforesaid amino acid sequences of this paragraph or a sequence at least 85%, in a further embodiment at least 90%, in a further embodiment at least 95%, in a further embodiment at least 98%, identical thereto. In an embodiment, the immunity polypeptide is comprised in a fusion polypeptide, said fusion polypeptide in an in an embodiment further comprising a binding agent.

Thus, the present invention also relates to a fusion polypeptide according to claim 12; further disclosure relates to a fusion polypeptide comprising a Colicin polypeptide as specified herein or an immunity polypeptide as specified herein and a binding agent as specified herein above.

In an embodiment, a complex of any of the aforesaid Colicin polypeptides with any of the aforesaid immunity polypeptides has a K_{D} value of at most 10⁻⁹ M, in an embodiment at most 10⁻¹⁰ M, in a further embodiment at most 10⁻¹¹ M, in a further embodiment at most 10⁻¹² M, in a further embodiment at most 10⁻¹³ M, in a further embodiment at most 10⁻¹⁴ M. In a further embodiment, a complex of any of the aforesaid Colicin polypeptides with the wildtype G. thermoglucosidasius immunity polypeptide, and/or a complex of any of the aforesaid immunity polypeptides with the wildtype G. thermoglucosidasius Colicin polypeptide has a K_{D} value of at most 10⁻⁹ M, in an embodiment at most 10⁻¹⁰ M, in a further embodiment at most 10⁻¹¹ M, in a further embodiment at most 10⁻¹² M, in a further embodiment at most 10⁻¹³ M, in a further embodiment at most 10⁻¹⁴ M.

The present invention further relates to a polypeptide complex according to claim 13, further disclosure relates to a polypeptide complex comprising a first partner of an affinity pair (first affinity partner); and a second partner of the affinity pair (second affinity partner) wherein (i) said first affinity partner is a Colicin polypeptide as specified herein above and said second affinity partner is a polypeptide comprising the amino acid sequence of SEQ ID NO:2 or a sequence at least 50% identical thereto; or (ii) said first affinity partner is a polypeptide comprising the amino acid sequence of SEQ ID NO:1 or a sequence at least 50% identical thereto and said second affinity partner is an immunity polypeptide as specified herein above.

The present invention also relates to a polynucleotide according to claim 14, further disclosure relates to a polynucleotide encoding a Colicin polypeptide as specified herein, an immunity polypeptide as specified herein, and/or a fusion polypeptide as specified herein.

The term "polynucleotide" is known to the skilled person. As used herein, the term includes nucleic acid molecules comprising or consisting of a nucleic acid sequence or nucleic acid sequences as specified herein. The polynucleotide of the present invention shall be provided, in an embodiment, either as an isolated polynucleotide (i.e. isolated from its natural context) or in genetically modified form. The polynucleotide, in an embodiment, is DNA, including cDNA, or is RNA. The term encompasses single as well as double stranded polynucleotides. In an embodiment, the polynucleotide is a chimeric molecule, i.e., in an embodiment, comprises at least one nucleic acid sequence, in an embodiment of at least 20 bp, in a further embodiment at least 100 bp, heterologous to the residual nucleic acid sequences. Moreover, in an embodiment, comprised are also chemically modified polynucleotides including naturally occurring modified polynucleotides such as glycosylated or methylated polynucleotides or artificial modified one such as biotinylated polynucleotides.

The polynucleotide encodes a polypeptide specified elsewhere herein. In an embodiment, the polynucleotide encodes a polypeptide comprising, in an embodiment consisting of, the amino acid sequence of SEQ ID NO:3, 4, 13, or 14. Thus, in an embodiment, the polynucleotide comprises, in a further embodiment consists of, the nucleic acid sequence of SEQ ID NO: 18 or 19. It is to be understood that a polypeptide having an amino acid sequence as detailed herein may also be encoded, due to the degenerated genetic code, by more than one species of polynucleotide. Thus, in an embodiment the polynucleotide comprises, in an embodiment consists of, a nucleic acid sequence at least essentially identical to the nucleic acid sequence of SEQ ID NO:18 or 19. The polynucleotide either essentially consist of the aforementioned nucleic acid sequence or comprises the aforementioned nucleic acid sequences. In an embodiment, the polynucleotide is an expression construct.

The term "expression construct", as used herein, relates to a polynucleotide comprising a nucleic acid sequence encoding a polypeptide as specified herein, operatively linked to at least one expression control sequence causing transcription of the coding sequence to occur. In an embodiment, expression occurs in eukaryotic cells, prokaryotic cells, and/or or isolated fractions thereof, in an embodiment into a translatable mRNA. Regulatory sequences for bacterial expression constructs, in particular promoters, such as the lac promoter, are known in the art. Regulatory elements ensuring expression in eukaryotic cells, in an embodiment mammalian cells, are also known in the art. They, in an embodiment, comprise regulatory sequences ensuring initiation of transcription, in particular at least one promoter, and, optionally, poly-A signals ensuring termination of transcription and stabilization of the transcript. In an embodiment, regulatory are cell- or tissue-type specific and/or are inducible, e.g. by administration of specific inducers. Preferred constitutive promoters are e.g. the CMV-, SV40-, or RSV (Rous sarcoma virus)-promoter, CMV-enhancer, or SV40-enhancer. Additional regulatory elements may include transcriptional as well as translational enhancers. In an embodiment, the enhancer is a CMV enhancer.

In an embodiment, the polynucleotide is comprised in a vector. The term "vector", in an embodiment encompasses phage, plasmid, viral or retroviral vectors as well as artificial chromosomes, such as bacterial or yeast artificial chromosomes, including other autonomously replicating polynucleotides. Moreover, the term also relates to targeting constructs which allow for random or site-directed integration of the targeting construct into genomic DNA. Such targeting constructs, in an embodiment, comprise DNA of sufficient length for either homologous or heterologous recombination. The vector encompassing the polynucleotides as specified herein, in an embodiment, further comprises at least one selectable marker for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Viral vectors may be replication competent or replication defective. In the latter case, viral propagation generally will occur only in complementing hosts and/or cells. In a further embodiment, in the vector, the polynucleotide is operatively linked to expression control sequences as specified herein above. Preferred expression vectors are vectors derived from viruses such as retroviruses, in particular lentiviruses, vaccinia virus, adeno-associated virus, or herpes viruses which may be used for delivery of the polynucleotide or vector of the invention into a targeted cell population. Methods used to construct recombinant polynucleotides, expression constructs, or vectors are well known to those skilled from standard text books.

Further disclosure relates to a method of producing a Colicin polypeptide as specified herein, an immunity polypeptide as specified herein, and/or a fusion polypeptide as specified herein, said method comprising expressing a polynucleotide encoding said polypeptide or fusion polypeptide in a eukaryotic cell or comprising expressing a polynucleotide encoding said fusion polypeptide in a eukaryotic cell or a prokaryotic cell, in an embodiment a eukaryotic cell.

As used herein, the term "host cell" relates to any cell capable of producing a polypeptide or fusion polypeptide as specified; thus, the host cell in an embodiment is capable of receiving an expression polynucleotide and/or an expression vector as specified herein. In an embodiment, the host cell is a bacterial cell, in a further embodiment a cell of a common laboratory bacterial strain known in the art, in an embodiment an Escherichia strain, in particular an E. coli strain. In a further embodiment, the host cell is a eukaryotic cell, in an embodiment a fungal, e.g. yeast cell, or is an animal cell. In an embodiment, the host cell is an insect cell or a mammalian cell, in particular a human, mouse, or rat cell. In a further embodiment, the host cell is a mammalian cell, e.g. a 293 human embryonic kidney (HEK) cell.

The method of producing a polypeptide or fusion polypeptide comprises a step of expressing an expression polynucleotide and/or an expression vector as specified herein in a host cell. The term "expressing" a polynucleotide is understood by the skilled person. In an embodiment, the term comprises introducing an expression polypeptide and/or an expression vector into a host cell, wherein the expression control sequences are selected as to correspond to the host cell, i.e. in particular mammalian or mammalian virus expression control sequences are used for a mammalian host cell. The aforesaid applies to other host cell types mutatis mutandis. Introducing the expression polynucleotide and/or an expression vector may be achieved by any method deemed appropriate by the skilled person, in particular those specified herein above.

In an embodiment, the method comprises further steps. E.g., after introduction of the expression polynucleotide and/or the expression vector, a further step may comprise incubating the host cell under conditions suitable for expression of the expression construct. Also, one or more further steps may relate to harvesting and/or purifying the polypeptide or polypeptide from the cells and/or from the culture supernatant.

Further disclosure relates to a method of purifying a polypeptide of interest, comprising
(A) providing a fusion polypeptide comprising said polypeptide of interest and a first partner of an affinity pair (first affinity partner);
(B) contacting the fusion polypeptide of step (A) with a second partner of the affinity pair (second affinity partner) coupled to a solid surface; and
(C) removing polypeptides not bound to said solid surface, thereby purifying said polypeptide of interest,
wherein one of said first affinity partner and said second affinity partner is a polypeptide comprising the amino acid sequence of SEQ ID NO:1 or a sequence at least 50% identical thereto, and wherein the other of said first affinity partner and said second affinity partner is a polypeptide comprising the amino acid sequence of SEQ **ID** NO:2 or a sequence at least 50% identical thereto.

A "polypeptide of interest" may be, in principle, any polypeptide of which the skilled person may be desirous of purifying. Thus, the polypeptide of interest may in principle, be any polypeptide. **In** an embodiment, the polypeptide of interest comprises a binding agent as specified herein above.

The fusion polypeptide in step (A) may be provided in any form deemed appropriate by the skilled person. **In** an embodiment, the fusion polypeptide in step (A) is comprised in a mixture comprising a multitude of other polypeptides, in particular a host cell lysate, a host cell culture supernatant, or a partially purified preparation of the fusion polypeptide. **In** an embodiment, the fusion polypeptide is provided in a non-denaturing aqueous solution, in particular in one of the buffers specified herein in the Examples. In an embodiment, the buffer is selected so as to not negatively affect the activity of the polypeptide of interest.

The contacting in step (B) may in particular be performed by contacting the fusion polypeptide with a chromatography material as a solid surface, i.e., may be performed as an affinity chromatography.

Washing step (C) may be performed by any method deemed appropriate by the skilled person in dependence on the purification format selected. In an embodiment, the washing buffer is a non-denaturing aqueous solution as specified herein above.

The method may comprise further steps; in particular a further step (D) may relate to eluting the fusion polypeptide or a fragment thereof comprising the polypeptide of interest from said solid surface. Said eluting may be performed by applying a denaturing buffer to the preparation obtained in step (C), thereby releasing the fusion polypeptide from the solid surface. However, the fusion polypeptide may however also be designed to comprise a cleavable bond between the first affinity partner and the polypeptide of interest, e.g. a protease recognition site, such that the polypeptide of interest may be released from the solid surface by addition of a corresponding protease or by activation of a proteolytic activity comprised in the fusion polypeptide. Similarly, the polypeptide may be released by a transferase activity.

The present invention also relates to a kit according to claim 15; further disclosure relates to a kit comprising
(I) a Colicin polypeptide or a polynucleotide encoding the same; and/or
(II) an immunity polypeptide or a polynucleotide encoding the same;
comprised in a housing.

The term "kit", as used herein, refers to a collection of the aforementioned compounds, means or reagents of the present invention which may or may not be packaged together. The components of the kit may be comprised by separate vials (i.e. as a kit of separate parts) within the housing or provided in a single vial. As used herein, the term "housing" relates to a casing comprising the components as specified, in an embodiment enabling transport, in a further embodiment translocation, of the compound or compounds. Moreover, it is to be understood that the kit in an embodiment is to be used for practicing the methods referred to herein above; thus, the kit in an embodiment is an analytic kit, in a further embodiment a diagnostic kit, in an embodiment for determining an analyte specified herein above. In such case, the Colicin polypeptide and/or the immunity polypeptide in an embodiment is comprised in the kit as a binding compound. It is, in an embodiment, envisaged that all components are provided in a ready-to-use manner for practicing at least one of the methods referred to above. Further, the kit, in an embodiment, contains instructions for carrying out said methods. The instructions can be provided by a user's manual in paper or electronic form. In addition, the manual may comprise instructions for administration and/or dosage instructions for carrying out the aforementioned methods using the kit.

Further disclosure relates to a device comprising a solid surface coated with (i) a polypeptide comprising the amino acid sequence of SEQ ID NO:1 or a sequence at least 50% identical thereto, or (ii) with a polypeptide comprising the amino acid sequence of SEQ ID NO:2 or a sequence at least 50% identical thereto.

The term "device", as used herein, relates to any and all means providing a solid surface deemed appropriate by the skilled person for the coating as specified. Thus, the device may e.g. be a a multi-well plate, in an embodiment a microtiter plate, or a bead. However, also other devices may be envisaged, e.g. in an embodiment supports with flat surfaces such as slides, e.g. glass slides, in an embodiment functionalized plastic surfaces, e.g., as in dip stick or indicator strips, in an embodiment, latex beads, e.g. for use in an agglutination assay, and the like. In an embodiment, the device further comprises the other of (i) and (ii), in an embodiment in a complex and/or further comprising a detection compound and/or a specifying compound.

Further disclosure relates to a system comprising the device of the present invention and a means for determining the amount of detection compound and/or of specifying compound present in said device.

The term "system", as used herein, relates to a system of means comprising at least the aforementioned means operatively linked to each other as to allow the determination. Typical means for determining the amounts of a complex comprising an indicator, and means for carrying out the determination are disclosed above in connection with the methods of the invention. How to link the means in an operating manner will depend on the type of means included into the device. In an embodiment, the means are comprised by a single device. Said device may accordingly include (i) an analyzing unit for the measurement of the complex and a (ii) computer unit for processing the resulting data for evaluation. Typical means for detection are disclosed in connection with embodiments relating to the method of the invention above. In such a case, the means are operatively linked in that the user of the system brings together the result of the determination of the optically or/and electrochemically determinable property of an indicator due to the instructions and interpretations given in a manual, or said instructions and interpretations are comprised in an executable program code comprised in the device, such that, as a result of determination, an amount or concentration of analyte in the sample applied is output to the user. The person skilled in the art will realize how to link the means without further ado. Typical systems are those which can be applied without the particular knowledge of a specialized technician, e.g., test stripes or electronic devices which merely require loading with a sample. The results may be given as output of raw data which need interpretation by a technician. In an embodiment, the output of the device is, however, processed, i.e. evaluated, raw data, the interpretation of which does not require a technician. Further typical devices comprise the analyzing units/devices (e.g., biosensors, arrays, solid supports coupled to ligands specifically recognizing the peptide, Plasmon surface resonance devices, NMR spectrometers, mass-spectrometers etc.) or evaluation units/devices referred to above in accordance with the methods of the invention.

In view of the above, in a generic disclosure, the following embodiments, which do not define the invention, are particularly envisaged:
Embodiment 1: A method of determining an analyte in a sample, said method comprising
   (a) contacting said sample with (i) a binding compound binding to said analyte, said binding compound comprising a binding agent and a first partner of an affinity pair (first affinity partner); and (ii) a second partner of the affinity pair (second affinity partner) coupled to a solid surface, to an indicator, and/or to a second binding agent; and
   (b) determining said analyte based on complexes formed in step (a);
   wherein one of said first affinity partner and said second affinity partner is a polypeptide comprising the amino acid sequence of SEQ ID NO:1 or a sequence at least 50% identical thereto, and wherein the other of said first affinity partner and said second affinity partner is a polypeptide comprising the amino acid sequence of SEQ ID NO:2 or a sequence at least 50% identical thereto.
Embodiment 2: The method of embodiment 1, wherein said binding agent comprises an antibody, an aptamer, an anticalin, a Designed Ankyrin Repeat Protein, a receptor, or a fragment of one of the aforesaid having the activity of binding to the analyte, in an embodiment comprises an antibody or fragment thereof having the activity of binding to the analyte.
Embodiment 3: The method of embodiment 1 or 2, wherein said binding compound is a fusion polypeptide comprising a binding agent and said first affinity partner.
Embodiment 4: The method of any one of embodiments 1 to 3, wherein the first affinity partner is a polypeptide comprising the amino acid sequence of SEQ ID NO:1 or a sequence at least 50% identical thereto, and wherein the second affinity partner is a polypeptide comprising the amino acid sequence of SEQ ID NO:2 or a sequence at least 50% identical thereto.
Embodiment 5: The method of any one of embodiments 1 to 4, wherein the first affinity partner is a polypeptide comprising the amino acid sequence of SEQ ID NO:3, and/or wherein the second affinity partner is a polypeptide comprising the amino acid sequence of SEQ ID NO:4. Embodiment 6: The method of any one of embodiments 1 to 5, wherein said sample is a sample of a subject, in an embodiment a tissue sample or a sample of a bodily fluid.
Embodiment 7: The method of any one of embodiments 1 to 6, wherein said sample is a plasma, serum, or blood sample.
Embodiment 8: The method of any one of embodiments 1 to 7, wherein said binding compound is a capture compound and wherein said second affinity partner is bound to a solid surface in step (b).
Embodiment 9: The method of any one of embodiments 1 to 8, wherein said binding compound is a capture compound and wherein said second affinity partner is bound to a solid surface and to a further capture compound in step (b).
Embodiment 10: The method of any one of embodiments 1 to 9, wherein said method further comprises a step of contacting said sample with a detection compound and wherein step (b) comprises determining the amount of complexes comprising the detection compound bound to the solid surface.
Embodiment 11: The method of embodiment 10, wherein said detection compound binds specifically to said analyte.
Embodiment 12: The method of embodiment 10 or 11, wherein said complexes comprising the detection compound further comprise the analyte, the binding compound comprising, the first affinity partner, and the second affinity partner.
Embodiment 13: The method of any one of embodiments 1 to 9, wherein said method further comprises a step of contacting said sample with a specifying compound and wherein step (b) comprises determining the amount of complexes comprising the specifying compound bound to the solid surface.
Embodiment 14: The method of embodiment 13, wherein said specifying compound competes in binding to the binding compound with the analyte.
Embodiment 15: The method of embodiment 13 or 14, wherein said complexes comprising the specifying compound further comprise the binding agent, the first affinity partner, and the second affinity partner.
Embodiment 16: The method of any one of embodiments 1 to 15, wherein said binding compound is a detection compound and wherein said second affinity partner is bound to an indicator in step (b).
Embodiment 17: The method of any one of embodiments 1 to 16, wherein said binding compound is a detection compound and wherein said second affinity partner is bound to an indicator and to a further detection compound in step (b).
Embodiment 18: The method of any one of embodiments 1 to 17, wherein said binding compound comprises the amino acid sequence of SEQ ID NO:10 or 12, in an embodiment covalently connected via at least one disulfide bridge to a polypeptide comprising, in an embodiment consisting of, SEQ ID NO:11.
Embodiment 19: A polypeptide comprising an amino acid sequence as specified in SEQ ID NO:1 or a sequence at least 50% identical thereto, wherein the amino acid at the position corresponding to position 77 in SEQ ID NO:1 is not a histidine, in an embodiment is alanine, glycine, leucine, or isoleucine.
Embodiment 20: The polypeptide of embodiment 19, wherein said polypeptide comprises an amino acid sequence as specified in SEQ ID NO:16.
Embodiment 21: The polypeptide of embodiment 19 or 20, wherein the amino acid at the position corresponding to position 35 in SEQ ID NO:1 is not a cysteine, in an embodiment is alanine, serine, leucine, isoleucine, or glycine.
Embodiment 22: The polypeptide of any one of embodiments 19 to 21, wherein a complex of said polypeptide with a polypeptide comprising the amino acid sequence of SEQ ID NO:2 has a K_{D} value of at most 10⁻⁹ M, in an embodiment at most 10⁻¹⁰ M, in a further embodiment at most 10⁻¹¹ M, in a further embodiment at most 10⁻¹² M, in a further embodiment at most 10⁻¹³ M, in a further embodiment at most 10⁻¹⁴ M.
Embodiment 23: The polypeptide of any one of embodiments 19 to 22, wherein said polypeptide comprises an amino acid sequence as specified in SEQ ID NO:3.
Embodiment 24: A polypeptide comprising an amino acid sequence as specified in SEQ ID NO:2 or a sequence at least 50% identical thereto, wherein (i) the amino acid at the position corresponding to position 17 in SEQ ID NO:2 is not a cysteine, in an embodiment is alanine, serine, leucine, isoleucine, or glycine and/or (ii) said polypeptide further comprises at least one functional peptide.
Embodiment 25: The polypeptide of embodiment 24, wherein a complex of said polypeptide with a polypeptide comprising the amino acid sequence of SEQ ID NO:1 has a K_{D} value of at most 10⁻⁹ M, in an embodiment 10⁻¹⁰ M, in a further embodiment at most 10⁻¹¹ M, in a further embodiment at most 10⁻¹² M, in a further embodiment at most 10⁻¹³ M, in a further embodiment at most 10⁻¹⁴ M.
Embodiment 26: The polypeptide of embodiment 24 or 25, wherein said polypeptide comprises an amino acid sequence as specified in SEQ ID NO:4.
Embodiment 27: The polypeptide of any one of embodiments 19 to 23, wherein said polypeptide comprises an amino acid sequence as specified in said embodiment or a sequence at least 75%, in an embodiment at least 85%, in a further embodiment at least 90%, in a further embodiment at least 95%, in a further embodiment at least 98%, identical thereto.
Embodiment 28: The polypeptide according to any one of embodiments 19 to 27, wherein said polypeptide further comprises a G residue immediately preceding said amino acid sequence, in an embodiment further comprises the amino acid sequence MG immediately preceding said amino acid sequence.
Embodiment 29: The polypeptide according to any one of embodiments 19 to 28, wherein said amino acid sequence MG is the N-terminal sequence of said polypeptide.
Embodiment 30: The polypeptide according to any one of embodiments 19 to 29, wherein said polypeptide further comprises a transglutaminase peptide, in an embodiment comprising the amino acid sequence YRYRQ (SEQ ID NO:15).
Embodiment 31: The polypeptide according to any one of embodiments 19 to 30, wherein said polypeptide further comprises a tag peptide, in an embodiment comprising the amino acid sequence (His)6 (SEQ ID NO:5), in a further embodiment (His)8 (SEQ ID NO:6).
Embodiment 32: The polypeptide according to any one of embodiments 19 to 31, wherein said polypeptide further comprises at least one linker peptide.
Embodiment 33: The polypeptide according to any one of embodiments 19 to 32, wherein said polypeptide is thermostable for at least 5 min at a temperature of 60°C.
Embodiment 34: A fusion polypeptide comprising a polypeptide according to any one of embodiments 19 to 33 and a binding agent.
Embodiment 35: The fusion polypeptide of embodiment 34, wherein said fusion polypeptide comprises, in an embodiment consists of, the amino acid sequence of SEQ ID NO: 10 or 12.
Embodiment 36: A polypeptide complex comprising a first partner of an affinity pair (first affinity partner); and a second partner of the affinity pair (second affinity partner) wherein (i) said first affinity partner is a polypeptide according to embodiment 19 and said second affinity partner is a polypeptide comprising the amino acid sequence of SEQ ID NO:2 or a sequence at least 50% identical thereto; or (ii) said first affinity partner is a polypeptide comprising the amino acid sequence of SEQ ID NO:1 or a sequence at least 50% identical thereto and said second affinity partner is a polypeptide according to embodiment 24.
Embodiment 37: A polynucleotide encoding a polypeptide according to any one of embodiments 19 to 33, and/or a fusion polypeptide according to embodiment 34 or 35.
Embodiment 38: A method of producing a polypeptide according to any one of embodiments 19 to 33, and/or a fusion polypeptide according to embodiment 34 or 35, said method comprising expressing a polynucleotide encoding said polypeptide or fusion polypeptide in a eukaryotic host cell or comprising expressing a polynucleotide encoding said fusion polypeptide in a eukaryotic host cell or a prokaryotic host cell, in an embodiment a eukaryotic host cell.
Embodiment 39: The method of embodiment 38, wherein said eukaryotic host cell is a mammalian host cell, in an embodiment a human host cell, in a further embodiment a 293 human embryonic kidney (HEK) cell.
Embodiment 40: The method of embodiment 38 or 39, wherein said polynucleotide encoding said polypeptide is a polynucleotide according to embodiment 37.
Embodiment 41: A method of purifying a polypeptide of interest, comprising
   (A) providing a fusion polypeptide comprising said polypeptide of interest and a first partner of an affinity pair (first affinity partner);
   (B) contacting the fusion polypeptide of step (A) with a second partner of the affinity pair (second affinity partner) coupled to a solid surface; and
   (C) removing polypeptides not bound to said solid surface, thereby purifying said polypeptide of interest,
   wherein one of said first affinity partner and said second affinity partner is a polypeptide comprising the amino acid sequence of SEQ ID NO:1 or a sequence at least 50% identical thereto, and wherein the other of said first affinity partner and said second affinity partner is a polypeptide comprising the amino acid sequence of SEQ ID NO:2 or a sequence at least 50% identical thereto.
Embodiment 42: A kit comprising
   (I) a polypeptide according to any one of embodiments 19 to 23 and 27 to 33 (first affinity partner) or a polynucleotide encoding the same; and/or
   (II) a polypeptide according to any one of embodiments 24 to 33 (second affinity partner) or a polynucleotide encoding the same;
   comprised in a housing.
Embodiment 43: A device comprising a solid surface coated with (i) a polypeptide comprising the amino acid sequence of SEQ ID NO:1 or a sequence at least 50% identical thereto, or (ii) with a polypeptide comprising the amino acid sequence of SEQ ID NO:2 or a sequence at least 50% identical thereto.
Embodiment 44: The device of embodiment 43, wherein said device is a multi-well plate, in an embodiment a microtiter plate, or a bead.
Embodiment 45: The device of embodiment 43 or 44, further comprising the other of (i) and (ii), in an embodiment in a complex.
Embodiment 46: The device of any one of embodiments 43 to 45, further comprising a detection compound and/or a specifying compound.
Embodiment 47: A system comprising the device of any one of embodiments 43 to 46 and a means for determining the amount of detection compound and/or of specifying compound present in said device.
Embodiment 48. A method of determining at least two analytes in a sample, said method comprising
   (a) contacting said sample with (i) a first binding compound binding to a first analyte, said first binding compound comprising a first binding agent and a first partner of an affinity pair (first affinity partner); (ii) a second binding compound binding to a second analyte, said second binding compound comprising a second binding agent and a second partner of said affinity pair (second affinity partner); and (iii) a capture agent coupled to a solid surface, wherein said capture agent binds to at least one of said first binding compound and said second binding compound; and
   (b) determining said analyte based on the complexes formed in step (a);
   wherein one of said first affinity partner and said second affinity partner is a polypeptide comprising the amino acid sequence of SEQ ID NO:1 or a sequence at least 50% identical thereto, and wherein the other of said first affinity partner and said second affinity partner is a polypeptide comprising the amino acid sequence of SEQ ID NO:2 or a sequence at least 50% identical thereto.
Embodiment 49: The method of embodiment 48, wherein said capture agent binds to at least one of said first binding agent and said second binding agent.
Embodiment 50: The method of embodiment 48 or 49, wherein said capture agent binds to at least one of said first affinity partner and said second affinity partner.

### Figure Legends

Fig.1: Western Blot Analysis of the Fab <TnT>- GthColT protein after Ni-NTA purification; 1: size marker, 2: flow through, 3: wash fractions, 4-5: elution fractions.
Fig.2: Western Blot Analysis of the Fab <TnT>-GthIM protein after Ni-NTA purification; 1: flow through, 2-4: wash fractions, 4-6: elution fractions.
Fig.3: Anti-His Western Blot showing purification of *GthCol* M1: Novex^{™} Sharp Pre-stained Protein Standard. M2: MagicMark^{™} XP Western Protein Standard. Lane 1: reference solution. Lane 2: flow through. Lanes 3-4 represent the eluted peak fractions. The arrow indicates the 15.7 kDa *GthCol.*
Fig.4: SDS-PAGE showing the purification of *GthCol.* M: Novex^{™} Sharp Pre-stained Protein Standard. Lane 1: reference solution. Lane 2: flow through. Lanes 3-4 represent the eluted peak fractions. The arrow indicates the 15.7 kDa *GthCol.*
Fig.5: SDS-PAGE showing SEC purification of *GthIM.* M: Novex^{™} Sharp Pre-stained Protein Standard. Lane 1: reference solution. Lanes 2-10 eluted fractions. The arrow indicates the 10.5 kDa *GthIM.*
Fig.6: Anti-His Western Blot showing SEC purification of *GthIm.* M1: Novex^{™} Sharp Pre-stained Protein Standard. M2: MagicMark^{™} XP Western Protein Standard. Lane 1 reference. Lanes 2-10: elution fractions. The arrow indicates the 10.5 kDa binding protein.
Fig.7: DSC analysis of *GthIM. N=2.* Tm 79°C.
Fig.8: DSC analysis of *GthCol. N=2.* Tm 63 °C.
Fig.9: DSC analysis of *the Gth*IM/*GthCol complex 1:3 under 150 mM KCl. N=2.* Tm 96 °C.
Fig. 10: Schematic Biacore assay to investigate interactions and cross reactivities between *GthCol, GthIM , E.coli* IM-7, ColE-7.
Fig.11: Biacore sensorgrams comparing *E.coli* and *Geobacillus thermoglucosidasius Colicin*/*Immunity* Protein kinetic signatures, *Gth*IM/*Gth*Col interaction with superior kinetics.
Fig. 12: Schematic Biacore assay. The goal was to investigate the accessibility of TnT and *GthCol* to the surface displayed Fab <TnT>-GthIM.
Fig.13: Biacore sensorgram showing the high affinity 37 °C interaction of the antibody Fab fragment Fab <TnT> - *GthIM* with *GthCol.* The Fab fragment associated *GthIM* binding site is fully accessible.
Fig.14: Biacore sensorgram showing the Fab <TnT> - *GthIM* / TnT 37°C kinetics.
Fig.15: Schematic Biacore assay. Test for ternary complex formation between the surface-displayed Fab <TnT>-GthIM, TnT and IgG antibody clone <TnT> M-11-7-variant (see WO 2021/028309 A1).
Fig.16 : Complex formation for surface displayed Fab <TnT> - *GthIM,* binding (1) to a mixture of *GthCol* and TnT, followed by (2) sandwich forming IgG <TnT>-M-11-7 variant and (3) final complex dissociation phase. Grey arrows indicate the start and stop of the injections.
Fig.17: Schematic Biacore assay. Test for ternary complex formation between the surface displayed Fab <TnT>-GthIM, TnT and IgG antibody clone <TnT>- M-11-7 variant.
Fig.18: Biacore sensorgrams exemplary showing 30 nM injections of A.) Fab <IGF-1> - *GthIM* and B.) Fab <IGF-1> *- GthCol,* A and B each to surface displayed IgG <TnT>- 11-7 *-GthIM* **C.)** Fab <IGF-1> *- GthIM* and **D.)** Fab <IGF-1> *- GthCol,* C and D each to surface-displayed IgG <TnT>- 11-7 *-GthCol.* Positive response signals were only obtained where *GthCol* and *GthIM* domain fusions built high affinity complexes.
Fig.19: Biacore sensorgrams showing *GthCol*/*GthIm* fused <TnT> and < IGF-1> bispecific antibody complexes binding to their antigens TnT and IGF-1. **A)** IGF-1 binding to IgG <TnT>- 11-7 *-GthCol* / Fab <IGF-1> *- GthIM* complex; **B)** TnT binding to IgG <TnT>- 11-7 *-GthCol* / Fab <IGF-1> *- GthIM* complex; **C)** IGF-1 binding to IgG <TnT>- 11-7 *-GthIM* / Fab <IGF1> - GthCol complex; **D)** TnT binding to IgG <TnT>- 11-7 *-GthIM* / Fab <IGF-1> *- GthCol* complex.
Fig.20: Model of a *GthCol*/*GthIM* trimerized bispecific antibody. One IgG <TnT>- 11-7 - *GthIM* (ribbon) is depicted in close proximity with two Fab <IGF1> *- GthCol* (transparent surface view). Via the *GthCol*/*GthIM* binding modules a trimeric antibody is formed with two specificities versus IGF-1 and TnT.

The following Examples shall merely illustrate the invention. They shall not be construed, whatsoever, to limit the scope of the invention.

### Example 1: Production of GthCol and GthIm

### Example 1.1 Construction of expression plasmids

The DNA sequences of the *Geobacillus thermoglucosidasius* C-terminal Colicin DNase domain and its cognate immunity protein were identified in a 3-DM data mining and taken from UniProt (A0A1Y3Q1S7 and A0A178U4V9). The sequences of *GthCol* and *GthIm* were engineered as follows: An additional Glycine residue as first amino acid after the Start-Methionine was inserted into the polypeptide chain. For further purification and conjugation a Transglutaminase substrate tag (Q tag) and an octa Histidin tag were added to the C-terminus of the proteins via Glycine/Serine linkers. For *GthCol,* the cysteine at position 36 was exchanged for alanine (C36A). Histidine at position 78 was exchanged for alanine (H78A) to inactivate DNase activity. For *GthIm,* the cysteine at position 18 was exchanged for alanine (C18A).

All gene syntheses were designed for the pQE-80L plasmid (Qiagen) and ordered at GeneArt. *E. coli* expression vectors were generated by molecular cloning of the *GthCol* and *GthIm* fragments into plasmid pQE80-Kan, using the restriction enzymes *EcoRI-HF* and *HindIII-HF* (NEB). Transformation was performed with chemically competent *E. coli* XL1-Blue cells (Agilent) according to the manufacturer's protocol. Cells were spread out on Lysogeny broth (LB) agar plates containing 50 µg/mL Kanamycin for selecting successfully transformed cells. The plates were incubated overnight at 37°C. 5 mL LB cultures were inoculated with a single colony and grown overnight at 37°C. Plasmid DNA was isolated according to the Qiagen *Miniprep kit* and ultimately transformed in the *E. coli* expression strain BL21 (Agilent). Glycerol stocks for inoculation of later cultures were prepared by adding 20% Glycerol to the starter culture and immediate snap freezing in liquid nitrogen.

### Example 1.2: Protein Production in E.coli

250 mL overnight culture was used to inoculate 1.5 L culture of LB containing 50 µg/mL Kanamycin and the *lac* promoter was induced with 0.5 mM Isopropyl-β-D-thiogalactoside (IPTG) at an OD₆₀₀ of ~0.8. Expression was allowed to continue for 4 hours at 37°C. Cells were harvested by centrifugation at 6,000 g for 20 min at 4°C. Cell extracts were prepared by French press lysis at 1.5 kbar in 20 mM Tris, 0.5 M NaCl, 5% Glycerol, 20 mM Imidazole, pH 8. Lysates were cleared by centrifugation at 12,000 rpm for 30 min at 4°C. Protease inhibitors (Complete Protease Inhibitor Cocktail, Roche) and DNase (DNase I, Roche) were added, in order to prevent protein degradation and to reduce viscosity of the lysates. The supernatant was sterile-filtered (0.2 µm) and applied to an equilibrated Ni²⁺⁻NTA column (HisTrapHP,GE Healthcare) using an ÄKTA^{™} Pure chromatography system. Non-specific and unbound proteins were removed by washing the column with alternating 20 mM Tris, 1 M NaCl, 5% Glycerol, 50 mM Imidazole, pH 8 and 20 mM Tris, 5% Glycerol, 50 mM Imidazole, pH 8. Proteins were finally eluted with 5 CV (column volumes) 20 mM Tris, 0.5 M NaCl, 5% Glycerol, 250 mM Imidazole, pH 8. Protein containing fractions were pooled and further purified via size-exclusion chromatography (HiLoad 60/600 Superdex 75 pg, GE Healthcare). Prior to application, sample was concentrated to a final volume of ≤ 1 mL using ultracentrifugation columns (Amicon^{®} Ultra Centrifugal Filter Devices, Merck). The column was equilibrated and proteins were eluted with 1.5 CV 20 mM Tris, 150 mM NaCl, pH 8 at a flow rate of 1 mL/min. Desired peak fractions were pooled, concentrated and nitrogen frozen until further use. Purity of the proteins was confirmed by SDS-PAGE and Western Blot.

In another embodiment the protein expression was carried out in 6 L lab scale fermenter. Culture media used fermentation medium and it was performed at 30°C, feed with 75% glycerol to regulate the pO2-value between 50% and 30% for 22.5 h, until OD600 of 46 was reached. The cells were harvested and resuspended in buffer pH 7.4 (20 mM Na₂HPO₄, 500 mM NaCl, 5% glycerol, and 20 mM imidazole). Cell disruption was carried out in a high-pressure homogenizer at 1500 bars. The cellular extract was pretreated with 1-2% Polymin-G20 before enzyme purification.

### Example 1.3: Cell Culture production of antibody fusion proteins

Sequence analysis of variable heavy and light chains of monoclonal mouse IgG was performed by RACE PCR after mRNA isolation by using 5'/3' RACE kit 2nd Gen (Roche Diagnostics GmbH) and PCR primers as described in the literature (Doenecke et al. (1997), Leukemia 11:1787). The PCR amplicons were directly sequenced using the same primers after purification.

In the case of monoclonal antibody anti-TnT M-11-7 (see WO 2021/028309 A1), mouse variable coding regions were fused to human immunoglobulin gamma-1 heavy chain (sw_hum:IGG1_HUMAN), and immunoglobulin kappa constant (sw_hum:IGKC_HUMAN) coding regions, in order to obtain a chimeric mouse/human antibody.

Subsequently, either ColA0A (*Geobacillus thermoglucosidasius* Colicin, A0A1Y3Q1S7) C36A,H78A mutant or ColA0A_ImP (Bacillaceae bacteriocin immunity protein, A0A178U4V9) C18A mutant protein fragments were fused to the C-termini of all three heavy chain fragments.

For secretion of the Fab <IGF-1> Fab, see e.g. WO2012/150321, and Fab <TnT> M-11-7 fusion proteins, a modified serum albumin leader sequence was N-terminally attached. Gene synthesis including codon optimization was done at Geneart (Thermo Fisher Scientific). Subsequently, expression constructs were subcloned via SalI/BamHI into the eukaryotic expression vector pMIMT (Roche Molecular Biochemicals).

Following gene synthesis at Geneart, <TnT>-M-11-7 expression constructs were subcloned via BsmI in-frame to a variable heavy chain mouse leader sequence featuring an intron as previously described (Norderhaug et al. (1997), J. Immunol. Methods. 204:77-87).

Eukaryotic expression was under the control of a human cytomegalovirus immediate-early enhancer/promoter region followed by a bovine growth hormone polyadenylation signal.

For transient gene expression in human embryonic kidney (HEK) 293 cells, the FreeStyle^{™} 293-F expression system (Thermo Fischer Scientific) was used: Cultures at ~ 2 × 10⁶ cells/ml were transfected with the respective LC/HC expression plasmid pair at a concentration of 0.5-1 mg/l cell culture complexed by the 293-Free^{™} transfection reagent (Merck Millipore) according to the manufacturer's guidelines. Three hours post-transfection, the histone deacetylase inhibitor valproic acid was added to 4 mM to boost protein production (Backliwal et al. (2008) Biotechnology and Bioengineering 101:182). Each day, the culture was supplemented with 6% (v/v) of a soybean peptone hydrolysate-based feed. Seven days post-transfection, the culture supernatant was collected, cleared from cell debris by centrifugation and stored at -80°C for purification.

### Example 1.4: Purification of antibody fusion proteins

The binding proteins, *GthCol* (16 kDa) or *GthIm* (13 kDa), were connected via a flexible amino acid linker to the heavy chain of Fab <TnT>- M-11-7 A102W- Q-H6 (Fab <TnT>- *GthCol,* Fab <TnT>- *GthIM,* and Fab <IGF-1> fragments, respectively (SEQ ID Nos: 10 and 12, respectively). The Fab fragment fusion proteins were expressed and Ni²⁺⁻NTA purified from HEK 293 cell supernatants. The supernatant was sterile-filtered (0.2 µm) and medium was exchanged by 20 mM sodium phosphate, 500 mM NaCl, 20 mM Imidazole, pH 7.4. The sample was applied to an equilibrated Ni²⁺⁻NTA column (HisTrapHP, GE Healthcare) using an ÄKTA^{™} Pure chromatography system. Non-specific and unbound proteins were removed by washing the column with 5 CV of sample buffer. Proteins were finally eluted with 5 CV 20 mM sodium phosphate, 0.5 M NaCl, 500 mM Imidazole, pH 7.4. Desired peak fractions were pooled and buffer was exchanged to 50 mM potassium phosphate, 150 mM KCl, pH 7.4 (Amicon^{®} Ultra Centrifugal Filter Devices, Merck). Protein solutions were nitrogen frozen until further use. Purity of the proteins was confirmed by SDS-PAGE and Western Blot.

### Example 1.5: SDS-PAGE and Western Blot analysis

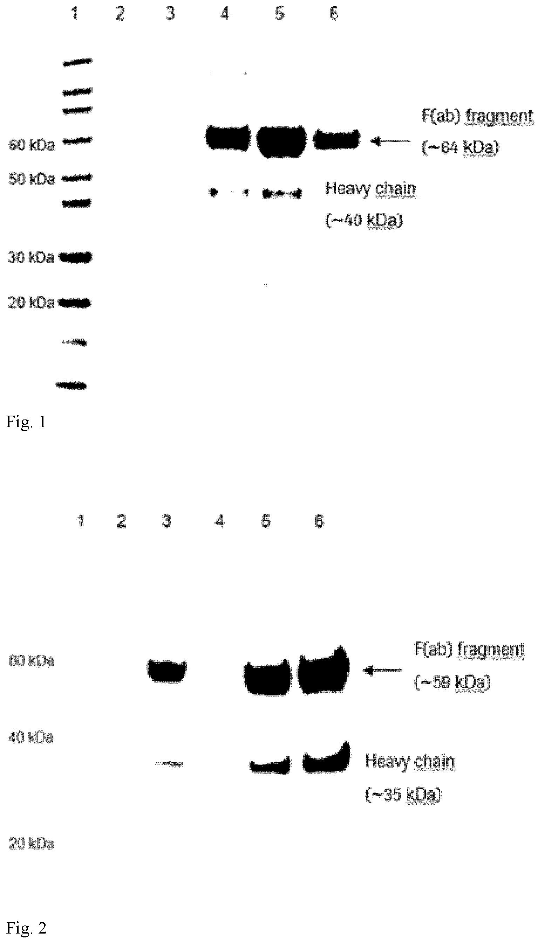

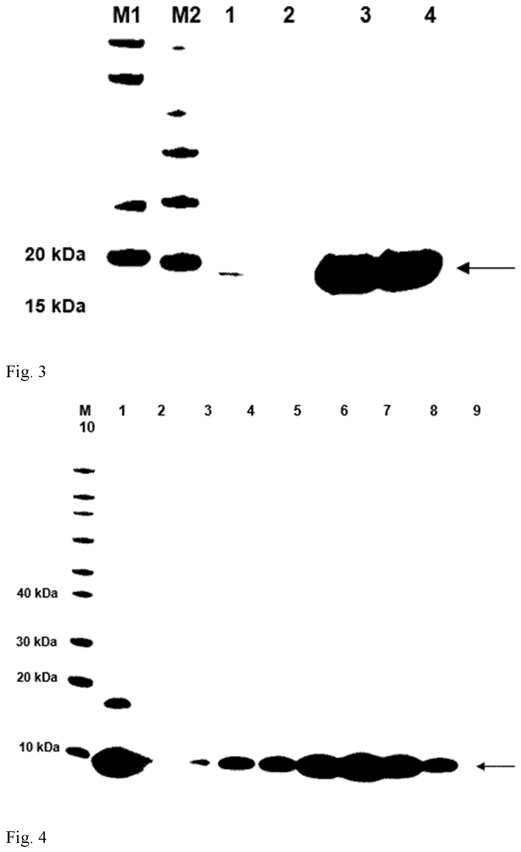

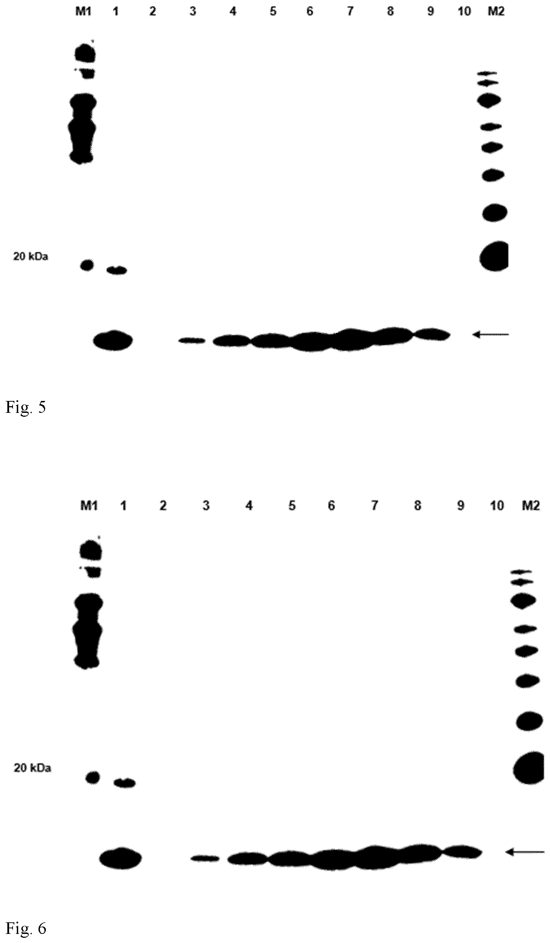

SDS-PAGE analyses were performed under reducing conditions according to standard protocols using NuPAGE^{®} Bis-Tris 4-12% gels (Thermo Fisher). Immunoblots on nitrocellulose membranes (iBlot^{™} Gel transfer stack nitrocellulose, midi/mini, Thermo Fisher) were carried out according to the manufacturer's instructions using anti-His-Peroxidase (Roche) for detection. The blots were visualized with Lumi-LightPLUS Western blotting substrate (Roche). Results are shown in Figures 1 to 6.

### Example 1.6: Protein stability determination by Differential Screening Calorimetry (DSC)

The melting temperature (Tm) of the proteins were determined by Microcal-VP-DSC (Malvern Panalytical). The DSC approach implemented duplicates of each sample with concentration of 1 mg/mL in 50 mM KH₂PO₄, 150 mM KCl, pH 7.4, using a heating rate of 1.5°C/min from 20°C to 120°C. The data analysis was performed with Origin (peak analysis of second derivative of Cp raw data).

### Example 1.7: Protein stability determination by Differential Scanning Fluorimetry (DSF)

Thermal unfolding experiments were performed with the Prometheus NT.Plex. The fluorescence intensity and the fluorescence maximum depends on the local tryptophan environment in the structure of the respective proteins. Therefore, the ratio of the fluorescence intensities at 350 nm and 330 nm is suitable to detect changes in protein structures, for example due to protein unfolding. The samples were measured in triplicates. 2 mg/ml *E.coli* Col-E7, 2.7 mg/ml *E.coli* IM-7, 0.25 mg/ml GthCol and 0.7 mg/ml GthIM were measured in 50 mM KH₂PO₄, 150 mM KCl, pH 7.4. The temperature was ramped from 20°C to 90°C. The fluorescence ratio 350 nm / 330 nm was determined.

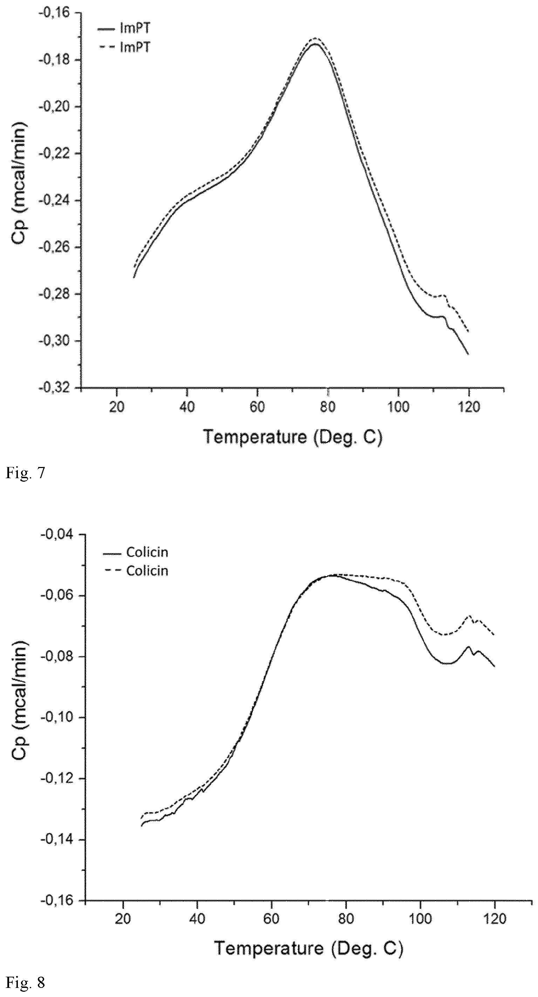

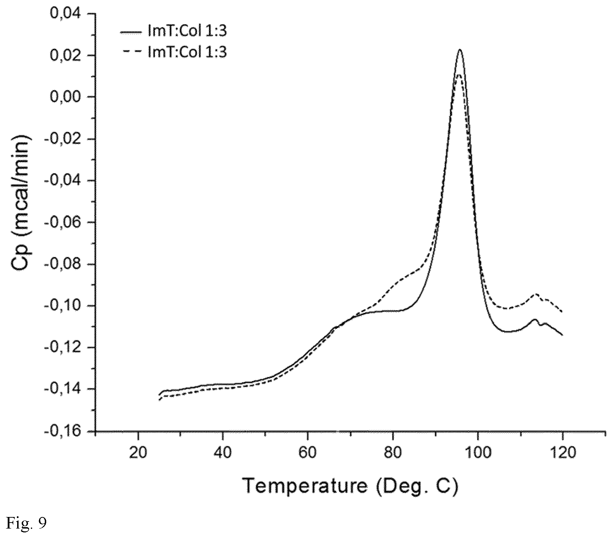

Results for DSF and DSC measurements are shown in Figs. 7 to 9 and Tab.1.

**Tab.1: Tm values determined with DSC and DSF. In both methods the new GthCol and GthIM binding modules show higher thermal stability when compared to the known E.coli ColE-7 and IM-7.**

| DSC stability | | | DSF stability | |
|---|---|---|---|---|
| | Tm [°C] | | | Tm [°C] |
| *Gth* Col | 63 | | *Gth* Col | 63 |
| *Gth* IM | 79 | | *Gth* IM | 68 |
| *Gth* Col *Gth* IM complex | 96 | | *ColE7* | 57 |
| | | | IM-7 | 39 |

The DSC melting experiments showed, that the Tm values of *GthIM, GthCol* and especially the *Gth*IM/*GthCol* complex are sufficiently stable for robust biotechnological applications, even under 150 mM KCl high salt conditions.

### Example 2: Interaction Analysis of E.coli IM7/ ColE, GthIM/GthCol, IM7/ GthCol and GthIM/ ColE and multi-specific constructs

### Example 2.1: BIAcore experiments

Kinetic investigations for *E. coli* Im7/ColE-7 respectively *Geobacillus thermoglucosidasius Gth*IM/*Gth*Col were performed at 37 °C using the BIAcore T200 instrument from former GE Healthcare. The goal was to investigate the affinity and specificity between *GthIM*/*GthCol* and IM7/ColE-7. Enzymatically singly biotinylated IM-7 (15 kDa) respectively singly biotinylated *GthIM* (13 kDa) were immobilized on a Streptavidin sensor surface and ColE-7(18 kDa) and *GthCol* (16 kDa) were used as analytes in solution. HBS-ET (10 mM HEPES, 150 mM NaCl, pH 7.4, 3 mM EDTA, 0.05 % (w/v) Tween20) was used as running and sample buffer.

A Biacore Streptavidin Series S sensor (Cat.No. BR-1005-31; Lot # 10271085) was mounted to the instrument and pretreated according to the manufacturer's instructions. Biotinylated IM-7 was injected on flow cell 2 using a concentration c = 5 nM with 100 seconds contact time and a flow rate 10 µL/ minutes. A ligand density of 204 Resonance Units (RU) was obtained for Im7. Free SA-binding sites were blocked subsequently to the Im7 immobilization: Amino-PEO-Biotin was injected using a concentration c = 50 µM with 3 minutes contact, flow rate 30 µl/min on flow cells 1 and 2; Flow cell 1 was used as reference for kinetic investigations. In another experiment biotinylated *GthIM* (13 kDa) was immobilized on a second SA-sensor as describes above, 139 RU ligand density on flow cell 3 was obtained for *GthIM.*

The kinetic characterization was performed using the single cycle kinetic method. 5 consecutive ColE-7 (18 kDa) or *GthCol* (16 kDa) concentrations from 1.1 nM to 90 nM were injected at 60 µL/min for 5 min association time. The dissociation phase was monitored for 1 hour after the final injection. Buffer injections served as a reference. The data were evaluated using double referencing to correct for bulk effects and systematic noise. The association rate constant *kₐ*[M⁻¹s⁻¹] and the dissociation rate constant *k_{d}* [s⁻¹] were evaluated using a Langmuir 1:1 fit model according to the BIAcore^{™} T200 Evaluation SW V 3.2 from GE Healthcare. Complex half-life (min) was calculated according to the formula ln(2)/60**k_{d}*.

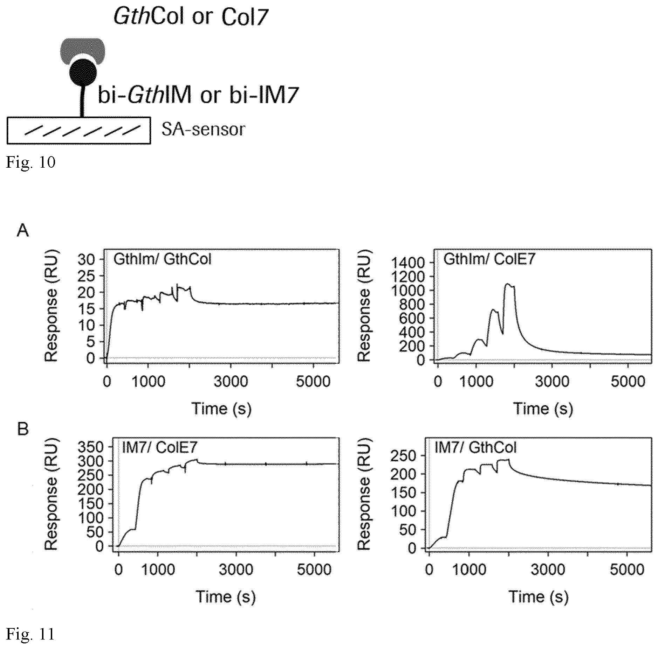

The resulting affinity was calculated with the formula: K_{D} = k_{d} / kₐ [M]. The Molar Ratio, the binding stoichiometry was calculated with the formula: MW (Im) / MW (Col) * Rₘₐₓ exp. (Col) / Ligand Density (Tm). A schematic overview is provided in Fig. 10, Results are shown in Fig. 11 and Tab. 2.

The binding signature for *Gth*Im/*Gth*Col shows fast complex formation with the association rate constant *kₐ* > 5.0E+07 M⁻¹s⁻¹. The complex dissociation is out of the instrument specifications *k_{d} <* 1.0E-05 s⁻¹. *GthIM* saturation is achieved at 3 nM *GthCol.* The *Gth*IM/*Gth*Col complex is highly stable at 37°C. The apparent affinity *K_{D}* < 2.0 E-13 M⁻¹ is clearly out of the instrument's specification. The Molar Ratio 1.1 indicates a 1:1 binding stoichiometry. The binding signature for *Gth*IM/ ColE7 shows a fast on-/off-profile with the association rate constant *kₐ >* 1.0E+07 M⁻¹s⁻¹ and a complex half-life time t/2-diss < 1 minute. The affinity *K_{D} =* 13 nM. *GthIM* unspecifically interacts with ColE7 approximately with 65000-fold less affinity when compared to *Gth*IM/*Gth*Col kinetics.

The IM7/ColE7 kinetic profile shows a fast complex formation into saturation with the association rate constant *kₐ* 1.8E+06 M⁻¹s⁻¹. The slow dissociation rate constant (*k_{d} <* 1.0E-05 s⁻¹) results in the complex half-life t/2-diss > 1155 minutes; The affinity *K_{D}* = 5.7 E-12 M⁻¹. The obtained constants are out of the instrument specification. The Molar Ratio 1.2 indicates 1:1 binding stoichiometry. The *Gth*Im/*Gth*Col interaction is at least 30-fold stronger than the IM7/ColE7 interaction. The binding signature for IM7/ *GthCol* shows a fast complex formation velocity with the association rate constant *kₐ* 1.2E+06 M⁻¹s⁻¹. The dissociation (*k_{d}* 9.2E-05 s⁻¹) results in the complex-half-life t/2-diss 126 minutes, the affinity *K_{D}* = 74 pM.

**Tab.2: Summary of kinetics with quantification where possible. GthIM/GthCol kinetics show higher affinity when compared to E.coli IM-7/ ColE-7 and all other combinations. No GthIM/GthCol complex dissociation was detectable within 1 hour. GthIM interacts with ColE-7 with strongly reduced complex stability. IM-7 interacts with GthCol with lowered complex stability. All interactions obey to a 1:1 binding stoichiometry as expected.**

| Complex | kₐ [M⁻¹s⁻¹] | k_{d} [s⁻¹] | K_{D} [M] | MR |
|---|---|---|---|---|
| *Gth*IM/*Gth*Col | > 5.0E+07 | < 1.0E-05 | < 2.0 E-13 | 1,1 |
| IM-7/ ColE-7 | 1,80E+06 | < 1.0E-05 | <5.7 E-12 | 1,2 |
| *Gth*IM/ ColE-7 | 1,00E+07 | 1,00E-01 | 1,30E-08 | 1,2 |
| IM-7/ GthCol | 1,20E+06 | 9,20E-05 | 7,40E-11 | 1,1 |

### Example 2.2: Interaction analyses of Fab <TnT>-GthCol with GthIM and TnT

In this experiment the goal was to investigate the antigen accessibility of Fab <TnT>-GthCol (64 kDa) by its cognate antigen TnT (37 kDa) and the *GthIM* (13 kDa) binding domain.

A Biacore CM5 Series S sensor (Cat. No.BR-29-1496-03, Lot # 10281824) was mounted to the instrument. Polyclonal Ab<M-IgG F(ab')₂>R (Jackson Immuno Research, Cat.No. #315-005-047, Lot #107797) was immobilized as capture system according to the to the manufacturer's instructions with up to 9829 RU. The system and sample buffer was HBS-EP (10 mM HEPES, 150 mM NaCl, 1 mM EDTA, 0.05 % (w/v) P20, pH 7.4). Flow cells 2, 3, 4 were used for the interaction measurements and flow cell 1 served as reference. Fab <TnT>-GthIM (59 kDa) was diluted in steps of 10 nM, 1 nM and 0.3 nM and were injected at 5 µL/min for 3 minutes. The Fab <TnT>-GthIM Capture Level (CL) in resonance units [RU] was monitored.

Increasing *GthCol* (16 kDa) concentrations 1.1 nM to 90 nM were injected at 60 µL/min in duplicates to the surface displayed <TnT>-Fab-*Gth*IM. The association phase was monitored for 5 min and the dissociation phase was monitored for 10 min respectively for 1 hour at 3.3 nM.

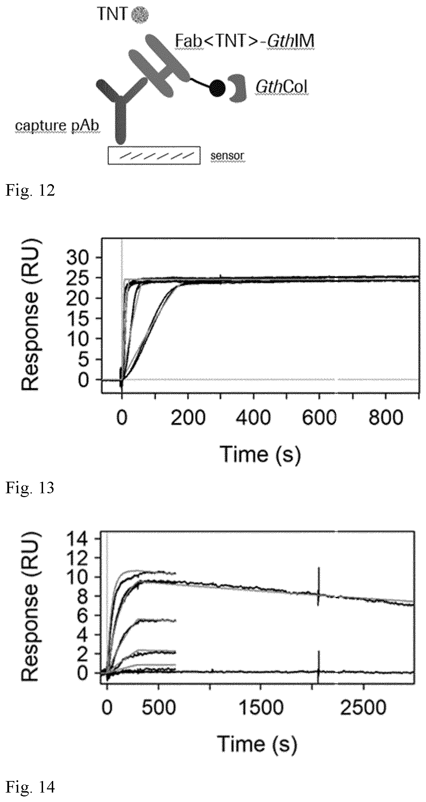

Increasing TnT (37 kDa, Roche in house) concentrations from 0.4 nM to 30 nM were injected at 60 µL/min with a 10 nM duplicate to the surface displayed Fab <TnT>-GthIM (59 kDa). The association phase was monitored for 5 min and the dissociation phase was monitored for 10 minutes respectively for 1 hour for the 10 nM concentration. After each cycle the Fab <TnT>-*GthIM* was regenerated from the capture system by an injection of 100 mM HCl at a flow rate 20 µL/min for 3 minutes. A schematic overview is provided in Fig. 12, Results are shown in Fig. 13.

Fab <TnT> - *GthIm* binds to the analyte *GthCol* with fast complex formation into saturation. Within 1 hour no dissociation was observed. The association rate constant was quantified with *kₐ* 9.9E+06 M-1s-1, the apparent dissociation (*k_{d} <* 1.0E-05 s-1 ) was out of the instrument's specifications. The affinity is apparently *K_{D} <* 1.0E-11 M. The Molar Ratio 1.0 indicates a 1:1 binding stoichiometry.

### Example 2.3: Fab <TnT> - GthIM binding Troponin T (TnT)

Fab <TnT> - *GthIM* binds TnT with a fast complex formation with the association rate constant *kₐ* 7.6E+05 M⁻¹s⁻¹. The dissociation rate constant *k_{d}* 9.2E-05 s⁻¹ results in the complex-half-life t/2-diss 125 minutes. The resulting affinity *K_{D}* is 121 pM. The Molar Ratio = 0.7 indicates a 1:1 binding stoichiometry. Fig. 14 and Tab.3 show the results.

**Tab.3: summary with 37°C kinetics for Fab <TnT> - GthIM complex formations. The Fab <TnT> - GthIM construct is fully accessible for the TnT antigen as well as for the GthCol binding domain. No steric hindrance is induced by the engineering of the Fab fragment with a linked GthIM domain.**

| Complex | kₐ [M⁻¹s⁻¹] | k_{d} [s⁻¹] | K_{D} [M] | MR |
|---|---|---|---|---|
| *Fab <TnT> -GthIM*/*GthCol* | 9,90E+06 | < 1.0E-05 | < 1.0E-11 | 1 |
| *Fab <TnT> -GthIM*/*TnT* | 7,60E+05 | 9,20E-05 | 1,21E-10 | 0,7 |

### Example 2.4: Ternary immune complex formation on TnT

In this experiment, the goal was to investigate accessibilities of the respective binding partners.

The capture system was used as described in the previous experiment. 1 nM Fab <TnT>-GthIM (59 kDa) were captured for 2 min at 5 µL/min. Free capture system binding sites were blocked with 1 µM mouse IgG Fab' fragment not recognizing TnT (37 kDa) or *GthCol* (16 kDa).

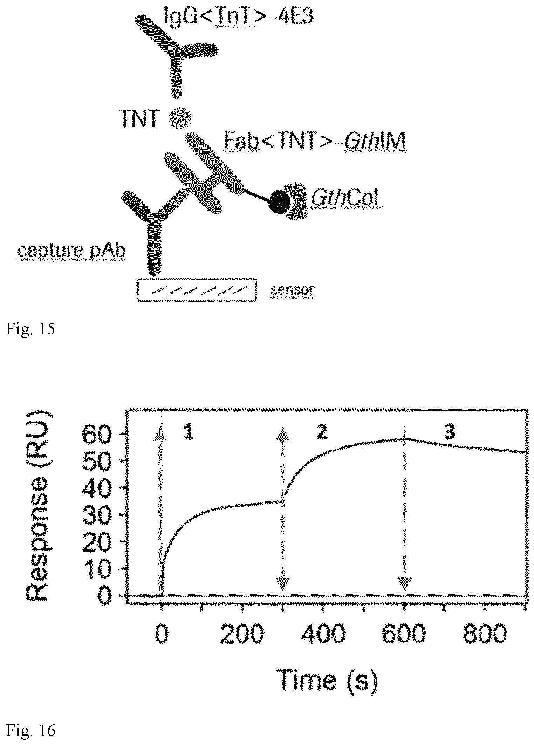

Two consecutive injections, using the dual injections function, were performed. 1^{st} a mixture of TnT (37 kDa) and Col (16 kDa) were offered for 5 minutes contact time to the surface displayed Fab <TnT>-GthIM, each with a concentration of 30 nM at a flow rate 20 µL/min. In a 2^{nd} injection 50 nM IgG antibody <TnT>-M-11-7 variant (160 kDa) was injected for 5 min at 20 µl/min. Regeneration after each cycle was performed as described. A schematic overview is provided in Fig. 15, Results are shown in Fig. 16.

*GthCol* and TnT binding sites are accessible. Further experiments (data not shown) demonstrated that Colicin and TnT independently bind from each other, demonstrated via permuted injection order. When offered as a mixture, both targets bind, resulting in the expected additive response. The IgG <TnT>-M-11-7 variant recognizes a TnT epitope different from Fab <TnT> - *GthIM.* The *GthIM* binding domain does not interfere with the antigen sandwich formation.

### Example 2.5: GthCol/GthIM mediated complexation of bispecific antibodies constructs

In this experiment, the goal was to investigate the ability to form binding active bispecific antibody formats.

Kinetic investigations were performed at 37°C using the BIAcore T200 instrument from GE Healthcare. mAb IgG <TnT>rH-M-11-7-*Gth*Col (173 kDa) respectively IgG mAb<TnT>rH-M-11-7-*Gth*IM (163 kDa) were captured on a CM5 series S sensor surface, <IGF1>-Fab-*GthCol* (64 kDa) or Fab <IGF1>-GthIM (59 kDa) were used as analytes in solution. System and sample buffer HBS-ET (10 mM HEPES, 150 mM NaCl, pH 7.4, 3 mM EDTA, 0.05 % (w/v) Tween20). A CM5 Series S sensor (Cat. No.BR-29-1496-03, Lot # 10286865) was mounted to the instrument. A monoclonal mAb<h-Fc-pan>M-R10Z8E9 (Roche in-house, Lot #2) was immobilized at 14509 RU on the sensor surface as capture system. The antibody was amine coupled using the EDC/NHS-chemistry according to the manufacturer's instructions.

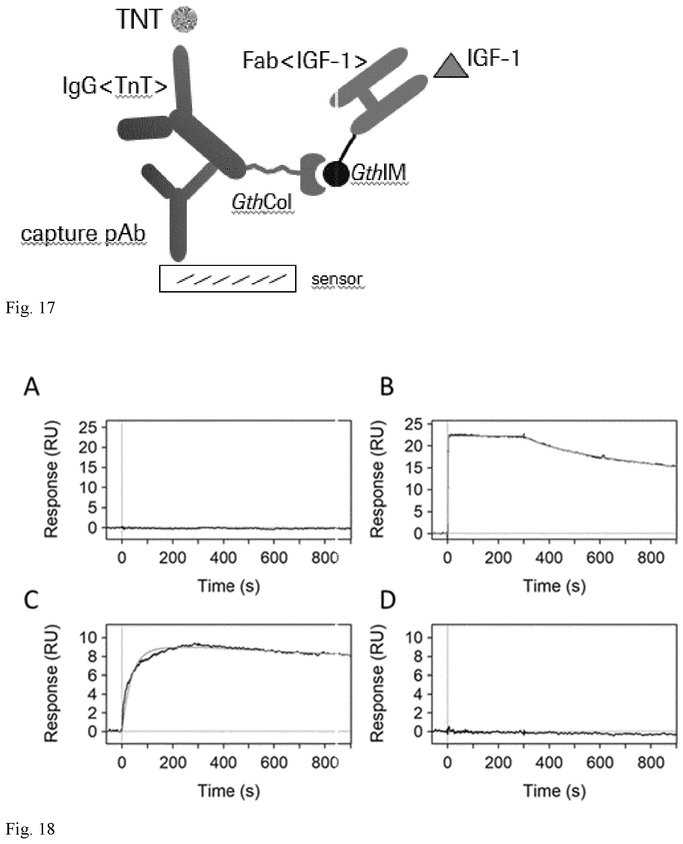

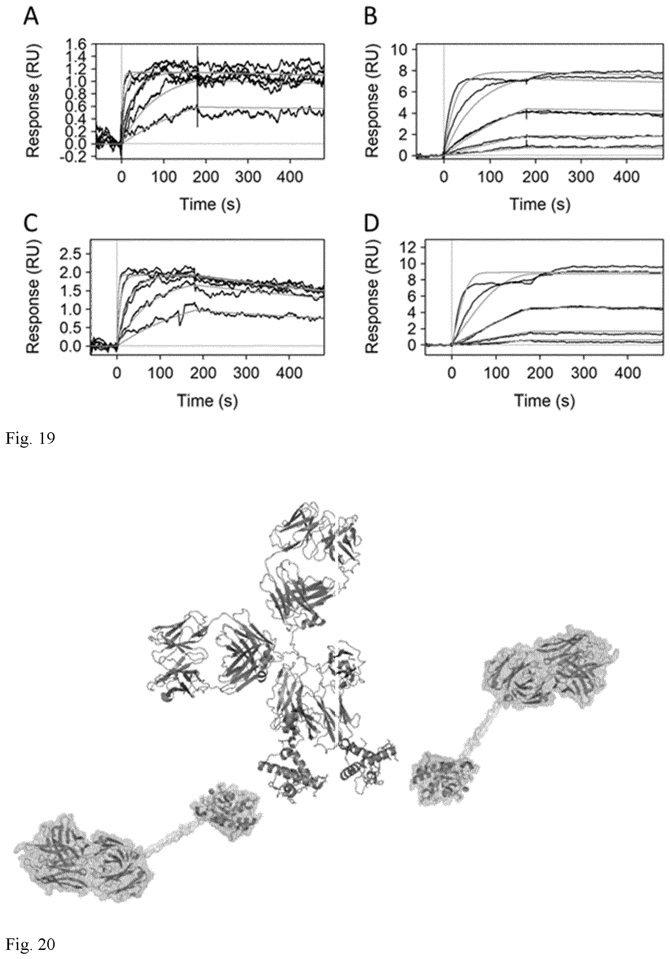

Flow cells 2, 3, 4 were used for the measurements and flow cell 1 served as reference. 0.5 nM of the respective antibodies were captured at 5 µL/min for 2 minutes on different flow cells. pAb<->H-IgG was injected as reference on flow cell 1. The Capture Levels (CL) in resonance units [RU] were monitored. 30 nM analytes <IGF1>-Fab-*Gth*Col or <IGF1>-Fab-GthIM were injected to the surface displayed mAb<TnT>rH-M-11-7-*Gth*Col-IgG respectively mAb<TnT>rH-M-11-7-*Gth*IM-IgG at 30 µL/min. To obtain the kinetic, an analyte concentration series from 0.04 nM to 30 nM, with duplicates for 1.1 nM were injected to the surface displayed antibodies at 30 µL/min. The association phase was monitored for 5 minutes; the dissociation phase was monitored for 10 minutes. Regeneration from the capture system was performed with 10 mM NaOH at a flow rate 20 µL/min for 15 seconds, followed by an injection of 10mM Glycine pH 2.5 for 2 minutes. TnT and IGF-1 binding to the bispecific complexes mAb<TnT>rH-M-11-7-*Gth*Col // <IGF1>-Fab-GthIM and mAb<TnT>rH-M-11-7-*Gth*IM-IgG//<IGF1>-Fab-*Gth*Col were tested at 25 °C. A series of increasing TnT (37 kDa) concentrations 0.4 nM to 30 nM, with duplicate for 3.3 nM respectively IGF-1 (7.7 kDa) 1.1 nM to 30 nM with duplicate for 10 nM, were injected to the surface-displayed preformed bispecific complexes at 30 µL/min. The association phase was monitored for 3 minutes; the dissociation phase was monitored for 5 minutes. A schematic overview is provided in Fig. 17, Results are shown in Figs. 18 to 20 and Tabs. 4 and 5.

*GthCol* and *GthIM* binding domains can facilitate the formation of bispecific antibody constructs, consisting e.g. from two Fab <IGF-1> fragments connected via HC linkers to a single IgG <TnT>-M-11-7 antibody. Different *GthCol* or *GthIM* antibody fusion constructs should elucidate the specificity and the suitability of antibody *Gth* binding domain combinations.

Fab <IGF-1> *- GthIM* binds to the surface displayed IgG <TnT>- 11-7 *- GthCol* (see B.) The affinity is *K_{D} =* 40 pM. The Molar Ratio = 0.9 indicates a 1:1 binding stoichiometry. No detectable binding (A.) for Fab <IGF1> - *Gth*Col with captured IgG <TnT>- 11-7 *-GthCol.* Fab <IGF-1> - *GthCol* (C.) binds to the surface displayed IgG <TnT>- 11-7 *-GthIM* with fast complex formation *kₐ* 4.8E+07 M⁻¹s⁻¹, the dissociation *k_{d}* 1.1E-03 s⁻¹ results in the complex-half-life t/2-diss > 7 minutes. The affinity is *K_{D}* = 35 pM. The Molar Ratio 1.9 indicates a 2:1 binding stoichiometry. No detectable binding (D.) for Fab <IGF-1> - *GthIM* with captured IgG <TnT>- 11-7 *-GthIM.*

**Tab.4 : Bispecific antibody assembly kinetics. The optimal construct pairing is listed in line three (3). Two GthCol domain fused Fab fragments and one GthIM fused IgG was found best suited (MR 1.9) for the assembly of a functional 2:1 trimeric antibody construct. No interaction was observable with homologous combinations (-).**

| | Complex | kₐ [M⁻¹s⁻¹] | kₐ [ s⁻¹] | K_{D} [M] | MR |
|---|---|---|---|---|---|
| 1 | Fab <IGF-1> *- GthIM* / IgG <TnT>-M-11-7 *-GthIM* | - | - | - | - |
| 2 | Fab <IGF-1> *- GthIM* /IgG <TnT>-M-11-7 *-GthCol* | 3,30E+06 | 1,30E-04 | 4,00E-11 | 0,9 |
| 3 | Fab <IGF-1> - *Gth*Col / IgG <TnT>-M-11-7 *-GthIM* | 4,80E+07 | 1,10E-03 | 3,50E-11 | **1,9** |
| 4 | Fab <IGF-1> - *Gth*Col / IgG <TnT>-M-11-7 *-GthCol* | - | - | - | - |

The optimal combination to generate a bispecific antibody is **C.)** . Two Fabs <IGF-1> - *GthCol* interact with two binding domains of IgG <TnT>- 11-7 *-GthIM.* This is indicated by a Molar Ratio of 1.9, which resembles to a 2:1 binding stoichiometry.

Next, the antigen accessibilities were tested. IGF-1 binds to the IgG <TnT>- 11-7 *-GthCol* / Fab <IGF1> *- GthIM* complex with *kₐ* 3.7E+06 M⁻¹s⁻¹ and dissociation *k_{d}* 1.4E-04 s⁻¹, representing a complex-half-life time t/2-diss 83 minutes. The resulting IGF-1 affinity is *K_{D} =* 37 pM. The Molar Ratio 1.4 indicates a 1:1 stoichiometry.

IGF-1 binds to the IgG <TnT>- 11-7 *-GthIM* / Fab <IGF1> - *Gth*Col complex with *kₐ* 3.8E+06 M⁻¹s⁻¹ and the dissociation *k_{d}* 8.1E-04 s⁻¹, which results in the complex-half-life t/2-diss = 14 minutes, the affinity is *K_{D}* = 212 pM. The Molar Ratio = 1.1, indicating a 1:1 binding stoichiometry.

TnT binds to the bispecific complex IgG <TnT>- 11-7 *-Gth*Col / Fab <IGF1> - *GthIM* with *kₐ* 1.4E+06 M⁻¹s⁻ and *k_{d}* 1.4E-04 s⁻¹, representing a complex-half-life t/2-diss 84 minutes. The affinity is *K_{D}* 97 pM. The Molar Ratio = 1.2 indicates a 1:1 stoichiometry.

TnT binds to the IgG <TnT>- 11-7 *-Gth*IM / Fab <IGF1> - *Gth*Col complex with *kₐ* 3.6E+06 M⁻¹s⁻¹ and *k_{d}* 1.4E-04 s⁻¹ (t/2-diss 116 minutes), *K_{D}* = 28 pM, the Molar Ratio = 1.3 indicates a 1:1 stoichiometry.

**Tab.5 : summary of IGF-1 and TnT kinetics to differently combined <TnT>- 11-7 / Fab <IGF-1> constructs. All combinations are accessible for their respective antigens.**

| | Complex | kₐ [M⁻¹s⁻¹] | k_{d} [s⁻¹] | K_{D} [M] | MR |
|---|---|---|---|---|---|
| 1 | TNT/FAB<IGF-1-GthCol/IgG<TnT>-5D8-GthIM | 3.60E+06 | 1.40E-04 | 2.80E-11 | 1.3 |
| 2 | TNT/FAB<IGF-1-GthIM/IgG<TnT>-5D8-GthCol | 1.40E+06 | 1.40E-04 | 9.70E-11 | 1.2 |
| 3 | IGF-1/Fab<IGF-1>-GthIM/IgG<TnT>-5D8-GthCol | 3.70E+06 | 1.40E-04 | 3.70E-11 | 1.1 |
| 4 | IGF-1/Fab<IGF-1>-GthColl/IgG<TnT>-5D8-GthIM | 3.80E+06 | 8.10E-04 | 2.12E-10 | 1.1 |

The calculation of the binding stoichiometries shows, that one TnT (37 kDa) molecule binds to one IgG <TnT>- 11-7 with a stoichiometry of 1:1 and one IGF-1 molecule (7.7 kDa) binds to one Fab <IGF-1> with a stoichiometry of 1:1. That means all surface displayed IGF-1 binding sites are saturated. Both Fab <IGF-1> fragments are accessible. The IgG <TnT>- 11-7 binds only one TNT antigen.

### Example 3: Solid Phase Immunoassay Measurements

### Example 3.1: Elecsys ECL TnT measurements

A commercial cardiac TnT high sensitivity Elecsys kit (Roche Diagnostics GmbH, Elecsys TroponinT hs, Mat. 050927744 190, Lot 42906601) was modified by replacing the biotinylated immunospecifier in the kit by the antibody fragment Fab <TnT> Fab *-Gth*Col*.* The ruthenylated immunospecifier remained the same as applied in the commercial kit. In order to capture the Fab fragment on the paramagnetic beads, the biotinylated *GthIM* binding domain was immobilized on the paramagnetic particles. The test was performed in triplicates on an Elecsys instrument e411.

The Diluent Multi Assay (Mat. Nr. 03609987) was used to determine the blank reference value. The Troponin T hs CalSet (Mat. Nr. 0509275190) was used to determine TnT concentrations using the Calibrator 1 with 18 pg/ml TnT and Calibrator 2 with 4200 pg/ml TnT. A new reagent was established in R1 using 2 µg/ml biotinylated *GthIM* in PBS and 10.3 µg/ml <TnT> Fab - *Gth*Col in PBS. As a control 2 µg/ml *GthIM* in PBS and 10. 3 µg/ml <TnT> Fab *-Gth*Col in PBS were used. Another control omitted the Fab fragment and just used 2µg/ml biotinylated *GthIM* in PBS.

### Example 3.2: Theoretical example on Troponin T (TnT) measurement

In another setup, the detection of TnT in an isolated patient sample (e.g. serum or plasma) in an assay format applying a solid phase or solid surface (e.g. beads- or multi titer plate-based or Biacore formats) can be modified. This setup does not apply biotin and streptavidin. In this approach, the solid phase is coated directly with immunity protein, e.g. via absorption or chemical coating known to a person skilled in the art. The capture compound, i.e. an antibody or fragment thereof specifically binding Troponin T, is covalently coupled to Colicin (e.g. to *Gth*Col or ColE-7), so that when exposed to the Colicin-coated solid phase, the TnT-specific antibody can directly bind to the solid phase. The other steps, i.e. binding of the analyte to the capture compound and binding of the specifier conjugated to an indicator (detectable label), are performed in an analogue way as in described in the previous example or as known to a person skilled in the art for competitive assay formats.

In yet another setup of the example, the capture compound, i.e. an antibody or fragment thereof specifically binding Troponin T, is covalently coupled to immunity protein (e.g. to *GthIM* or IM-7), so that when exposed to the Colicin-protein-coated solid phase, the TnT-specific antibody can directly bind to the solid phase. The subsequent steps can be performed as described above.

### References:

Backliwal et al. (2008) Biotechnology and Bioengineering 101: 182
Cascales et al. (2007) Microbiol Mol Biol Rev 71(1):158
Doenecke et al. (1997), Leukemia 11:1787
Garinot-Schneider et al. (1996) J Mol Biol 260(5):731
Keeble et al. (2006) Biochemistry 45(10):3243
Kuhlmann et al. (2000) J Mol Biol 301:1163
Norderhaug et al. (1997), J. Immunol. Methods. 204:77
Wallis et al. 1995 Biochemistry 34(42):13743
WO 2017/100584 A1
WO 2021/028309 A1
WO2012/150321

## Claims

1. A method of determining an analyte in a sample, said method comprising
(a) contacting said sample with (i) a binding compound binding to said analyte, said binding compound comprising a binding agent and a first partner of an affinity pair (first affinity partner); and (ii) a second partner of the affinity pair (second affinity partner) coupled to a solid surface, to an indicator, and/or to a second binding agent; and
(b) determining said analyte based on complexes formed in step (a);
wherein one of said first affinity partner and said second affinity partner is a polypeptide comprising the amino acid sequence of SEQ ID NO:1 or a sequence at least 85% identical thereto, and wherein the other of said first affinity partner and said second affinity partner is a polypeptide comprising the amino acid sequence of SEQ ID NO:2 or a sequence at least 85% identical thereto.

2. The method of claim 1, wherein said binding agent comprises an antibody, an aptamer, an anticalin, a Designed Ankyrin Repeat Protein, a receptor, or a fragment of one of the aforesaid having the activity of binding to the analyte, in an embodiment comprises an antibody or fragment thereof having the activity of binding to the analyte.

3. The method of claim 1 or 2, wherein the first affinity partner is a polypeptide comprising the amino acid sequence of SEQ ID NO:3, in an embodiment of SEQ ID NO: 13, and/or wherein the second affinity partner is a polypeptide comprising the amino acid sequence of SEQ ID NO:4, in an embodiment of SEQ ID NO: 14.

4. The method of any one of claims 1 to 3, wherein said sample is a sample of a subject, in an embodiment a tissue sample or a sample of a bodily fluid, in a further embodiment is a plasma, serum, or blood sample.

5. The method of any one of claims 1 to 4, wherein said binding compound is a capture compound and wherein said second affinity partner is bound to a solid surface in step (b), in an embodiment wherein said binding compound is a capture compound and wherein said second affinity partner is bound to a solid surface and to a further capture compound in step (b).

6. The method of any one of claims 1 to 5, wherein said method further comprises a step of contacting said sample with a detection compound and wherein step (b) comprises determining the amount of complexes comprising the detection compound bound to the solid surface.

7. The method of any one of claims 1 to 5, wherein said method further comprises a step of contacting said sample with a specifying compound and wherein step (b) comprises determining the amount of complexes comprising the specifying compound bound to the solid surface.

8. The method of any one of claims 1 to 7, wherein said binding compound comprises the amino acid sequence of SEQ ID NO:10 or 12, in an embodiment covalently connected via at least one disulfide bridge to a polypeptide comprising, in an embodiment consisting of, SEQ ID NO: 11.

9. A polypeptide comprising the amino acid sequence as specified in SEQ ID NO:1 or a sequence at least 85% identical thereto, wherein the amino acid at the position corresponding to position 77 in SEQ ID NO:1 is not a histidine, in an embodiment is alanine, glycine, leucine, or isoleucine, in an embodiment wherein said polypeptide comprises the amino acid sequence as specified in SEQ ID NO:16.

10. A polypeptide comprising the amino acid sequence as specified in SEQ ID NO:2 or a sequence at least 85% identical thereto, wherein (i) the amino acid at the position corresponding to position 17 in SEQ ID NO:2 is not a cysteine, in an embodiment is alanine, serine, leucine, isoleucine, or glycine and/or (ii) said polypeptide further comprises at least one functional peptide.

11. The polypeptide according to claim 9 or 10, wherein (i) said polypeptide further comprises a G residue immediately preceding said amino acid sequence, in an embodiment further comprises the amino acid sequence MG immediately preceding said amino acid sequence; (ii) said polypeptide further comprises a transglutaminase peptide, in an embodiment comprising the amino acid sequence YRYRQ (SEQ ID NO:15); (iii) said polypeptide further comprises a tag peptide, in an embodiment comprising the amino acid sequence (His)6 (SEQ ID NO:5), in a further embodiment (His)8 (SEQ ID NO:6); and/or (iv) said polypeptide further comprises at least one linker peptide.

12. A fusion polypeptide comprising a polypeptide according to any one of claims 9 to 11 and a binding agent.

13. A polypeptide complex comprising a first partner of an affinity pair (first affinity partner); and a second partner of the affinity pair (second affinity partner) wherein (i) said first affinity partner is a polypeptide according to claim 9 and said second affinity partner is a polypeptide comprising the amino acid sequence of SEQ ID NO:2 or a sequence at least 85% identical thereto; or (ii) said first affinity partner is a polypeptide comprising the amino acid sequence of SEQ ID NO:1 or a sequence at least 85% identical thereto and said second affinity partner is a polypeptide according to claim 10.

14. A polynucleotide encoding a polypeptide according to any one of claims 9 to 11, and/or a fusion polypeptide according to claim 12.

15. A kit comprising
(I) a polypeptide according to claim 9 (first affinity partner) or a polynucleotide encoding the same; and/or
(II) a polypeptide according to claim 10 (second affinity partner) or a polynucleotide encoding the same;
comprised in a housing.

## Patentansprüche

1. Verfahren zur Bestimmung eines Analyten in einer Probe, wobei das Verfahren Folgendes umfasst:
(a) Inkontaktbringen der Probe mit (i) einer Bindungsverbindung, die an den Analyten bindet, wobei die Bindungsverbindung ein Bindemittel und einen ersten Partner eines Affinitätspaares (erster Affinitätspartner) umfasst; und (ii) einem zweiten Partner des Affinitätspaares (zweiter Affinitätspartner), der an eine feste Oberfläche, einen Indikator und/oder ein zweites Bindemittel gekoppelt ist; und
(b) Bestimmen des Analyten auf der Grundlage der in Schritt (a) gebildeten Komplexe;
wobei einer von dem ersten Affinitätspartner und dem zweiten Affinitätspartner ein Polypeptid ist, umfassend die Aminosäuresequenz von SEQ ID NO:1 oder eine Sequenz, die zu dieser zu mindestens 85 % identisch ist, und wobei der andere von dem ersten Affinitätspartner und dem zweiten Affinitätspartner ein Polypeptid ist, umfassend die Aminosäuresequenz von SEQ ID NO:2 oder eine Sequenz, die zu dieser zu mindestens 85 % identisch ist.

2. Verfahren nach Anspruch 1, wobei das Bindemittel einen Antikörper, ein Aptamer, ein Anticalin, ein Designed Ankyrin Repeat Protein, einen Rezeptor oder ein Fragment eines der vorgenannten mit der Aktivität der Bindung an den Analyten umfasst, in einer Ausführungsform einen Antikörper oder ein Fragment davon mit der Aktivität der Bindung an den Analyten umfasst.

3. Verfahren nach Anspruch 1 oder 2, wobei der erste Affinitätspartner ein Polypeptid ist, umfassend die Aminosäuresequenz von SEQ ID NO:3, in einer Ausführungsform von SEQ ID NO:13, und/oder wobei der zweite Affinitätspartner ein Polypeptid ist, umfassend die Aminosäuresequenz von SEQ ID NO:4, in einer Ausführungsform von SEQ ID NO:14.

4. Verfahren nach einem der Ansprüche 1 bis 3, wobei die Probe eine Probe eines Subjekts ist, in einer Ausführungsform eine Gewebeprobe oder eine Probe einer Körperflüssigkeit, in einer weiteren Ausführungsform eine Plasma-, Serum- oder Blutprobe.

5. Verfahren nach einem der Ansprüche 1 bis 4, wobei die Bindungsverbindung eine Einfangverbindung ist und wobei der zweite Affinitätspartner in Schritt (b) an eine feste Oberfläche gebunden wird, wobei in einer Ausführungsform die Bindungsverbindung eine Einfangverbindung ist und wobei der zweite Affinitätspartner in Schritt (b) an eine feste Oberfläche und an eine weitere Einfangverbindung gebunden wird.

6. Verfahren nach einem der Ansprüche 1 bis 5, wobei das Verfahren ferner einen Schritt des Inkontaktbringens der Probe mit einer Nachweisverbindung umfasst und wobei Schritt (b) das Bestimmen der Menge an Komplexen umfasst, die die an die feste Oberfläche gebundene Nachweisverbindung umfassen.

7. Verfahren nach einem der Ansprüche 1 bis 5, wobei das Verfahren ferner einen Schritt des Inkontaktbringens der Probe mit einer spezifizierenden Verbindung umfasst und wobei Schritt (b) das Bestimmen der Menge an Komplexen umfasst, die die an die feste Oberfläche gebundene spezifizierende Verbindung umfassen.

8. Verfahren nach einem der Ansprüche 1 bis 7, wobei die Bindungsverbindung die Aminosäuresequenz von SEQ ID NO:10 oder 12 umfasst, die in einer Ausführungsform über mindestens eine Disulfidbrücke kovalent an ein Polypeptid gebunden ist, das SEQ ID NO:11 umfasst, in einer Ausführungsform daraus besteht.

9. Polypeptid, das die in SEQ ID NO:1 angegebene Aminosäuresequenz oder eine zu dieser zu mindestens 85 % identische Sequenz umfasst, wobei die Aminosäure an der Position, die der Position 77 in SEQ ID NO:1 entspricht, kein Histidin ist, in einer Ausführungsform Alanin, Glycin, Leucin oder Isoleucin ist, wobei in einer Ausführungsform das Polypeptid die in SEQ ID NO:16 angegebene Aminosäuresequenz umfasst.

10. Polypeptid, das die in SEQ ID NO:2 angegebene Aminosäuresequenz oder eine zu dieser zu mindestens 85 % identische Sequenz umfasst, wobei (i) die Aminosäure an der Position, die der Position 17 in SEQ ID NO:2 entspricht, kein Cystein ist, in einer Ausführungsform Alanin, Serin, Leucin, Isoleucin oder Glycin ist und/oder (ii) das Polypeptid ferner mindestens ein funktionelles Peptid umfasst.

11. Polypeptid gemäß Anspruch 9 oder 10, wobei (i) das Polypeptid ferner einen G-Rest unmittelbar vor der Aminosäuresequenz umfasst, in einer Ausführungsform ferner die Aminosäuresequenz MG unmittelbar vor der Aminosäuresequenz umfasst; (ii) das Polypeptid ferner ein Transglutaminase-Peptid umfasst, in einer Ausführungsform umfassend die Aminosäuresequenz YRYRQ (SEQ ID NO:15); (iii) das Polypeptid ferner ein Tag-Peptid umfasst, in einer Ausführungsform umfassend die Aminosäuresequenz (His)6 (SEQ ID NO:5), in einer weiteren Ausführungsform (His)8 (SEQ ID NO:6); und/oder (iv) das Polypeptid ferner mindestens ein Linker-Peptid umfasst.

12. Fusionspolypeptid, umfassend ein Polypeptid gemäß einem der Ansprüche 9 bis 11 und ein Bindemittel.

13. Polypeptidkomplex, umfassend einen ersten Partner eines Affinitätspaares (erster Affinitätspartner) und einen zweiten Partner des Affinitätspaares (zweiter Affinitätspartner), wobei (i) der erste Affinitätspartner ein Polypeptid gemäß Anspruch 9 ist und der zweite Affinitätspartner ein Polypeptid ist, das die Aminosäuresequenz von SEQ ID NO:2 oder eine Sequenz umfasst, die zu dieser zu mindestens 85 % identisch ist; oder (ii) der erste Affinitätspartner ein Polypeptid ist, das die Aminosäuresequenz von SEQ ID NO:1 oder eine Sequenz umfasst, die zu dieser zu mindestens 85 % identisch ist, und der zweite Affinitätspartner ein Polypeptid gemäß Anspruch 10 ist.

14. Polynukleotid, das ein Polypeptid gemäß einem der Ansprüche 9 bis 11 und/oder ein Fusionspolypeptid gemäß Anspruch 12 kodiert.

15. Kit, umfassend:
(I) ein Polypeptid gemäß Anspruch 9 (erster Affinitätspartner) oder ein Polynukleotid, das dasselbe kodiert; und/oder
(II) ein Polypeptid gemäß Anspruch 10 (zweiter Affinitätspartner) oder ein Polynukleotid, das dasselbe kodiert;
enthalten in einem Gehäuse.

## Revendications

1. Procédé de détermination d'un analyte dans un échantillon, ledit procédé comprenant
(a) la mise en contact dudit échantillon avec (i) un composé de liaison se liant audit analyte, ledit composé de liaison comprenant un agent de liaison et un premier partenaire d'une paire d'affinité (premier partenaire d'affinité) ; et (ii) un second partenaire de la paire d'affinité (second partenaire d'affinité) couplé à une surface solide, à un indicateur et/ou à un second agent de liaison ; et
(b) la détermination dudit analyte en se basant sur les complexes formés à l'étape (a) ;
dans lequel un parmi ledit premier partenaire d'affinité et ledit second partenaire d'affinité est un polypeptide comprenant la séquence d'acides aminés de SEQ ID NO : 1 ou une séquence au moins identique à 85 % à celle-ci, et dans lequel l'autre parmi ledit premier partenaire d'affinité et ledit second partenaire d'affinité est un polypeptide comprenant la séquence d'acides aminés de SEQ ID NO : 2 ou une séquence au moins identique à 85 % à celle-ci.

2. Procédé selon la revendication 1, dans lequel ledit agent de liaison comprend un anticorps, un aptamère, une anticaline, une protéine de répétition ankyrine conçue, un récepteur, ou un fragment de l'un des précédents ayant l'activité de liaison à l'analyte, dans un mode de réalisation comprend un anticorps ou un fragment de celui-ci ayant l'activité de liaison à l'analyte.

3. Procédé selon la revendication 1 ou 2, dans lequel le premier partenaire d'affinité est un polypeptide comprenant la séquence d'acides aminés de SEQ ID NO : 3, dans un mode de réalisation de SEQ ID NO : 13, et/ou dans lequel le second partenaire d'affinité est un polypeptide comprenant la séquence d'acides aminés de SEQ ID NO : 4, dans un mode de réalisation de SEQ ID NO : 14.

4. Procédé selon l'une quelconque des revendications 1 à 3, dans lequel ledit échantillon est un échantillon d'un sujet, dans un mode de réalisation un échantillon de tissu ou un échantillon d'un fluide corporel, dans un mode de réalisation supplémentaire est un échantillon de plasma, de sérum ou de sang.

5. Procédé selon l'une quelconque des revendications 1 à 4, dans lequel ledit composé de liaison est un composé de capture et dans lequel ledit second partenaire d'affinité est lié à une surface solide dans l'étape (b), dans un mode de réalisation dans lequel ledit composé de liaison est un composé de capture et dans lequel ledit second partenaire d'affinité est lié à une surface solide et à un composé de capture supplémentaire à l'étape (b).

6. Procédé selon l'une quelconque des revendications 1 à 5, dans lequel ledit procédé comprend en outre une étape de mise en contact dudit échantillon avec un composé de détection et dans lequel l'étape (b) comprend la détermination de la quantité de complexes comprenant le composé de détection lié à la surface solide.

7. Procédé selon l'une quelconque des revendications 1 à 5, dans lequel ledit procédé comprend en outre une étape de mise en contact dudit échantillon avec un composé de spécification et dans lequel l'étape (b) comprend la détermination de la quantité de complexes comprenant le composé de spécification lié à la surface solide.

8. Procédé selon l'une quelconque des revendications 1 à 7, dans lequel ledit composé de liaison comprend la séquence d'acides aminés de SEQ ID NO : 10 ou 12, dans un mode de réalisation lié de manière covalente par l'intermédiaire d'au moins un pont disulfure à un polypeptide comprenant, dans un mode de réalisation constitué de, la SEQ ID NO : 11.

9. Polypeptide comprenant la séquence d'acides aminés telle que spécifiée dans la SEQ ID NO : 1 ou une séquence identique à au moins 85 % à celle-ci, dans lequel l'acide aminé au niveau de la position correspondant à la position 77 dans la SEQ ID NO : 1 n'est pas une histidine, dans un mode de réalisation est l'alanine, la glycine, la leucine ou l'isoleucine, dans un mode de réalisation dans lequel ledit polypeptide comprend la séquence d'acides aminés telle que spécifiée dans la SEQ ID NO : 16.

10. Polypeptide comprenant la séquence d'acides aminés telle que spécifiée dans la SEQ ID NO : 2 ou une séquence au moins identique à 85 % à celle-ci, dans lequel (i) l'acide aminé au niveau de la position correspondant à la position 17 dans la SEQ ID NO : 2 n'est pas une cystéine, dans un mode de réalisation est l'alanine, la sérine, la leucine, l'isoleucine ou la glycine et/ou (ii) ledit polypeptide comprend en outre au moins un peptide fonctionnel.

11. Polypeptide selon la revendication 9 ou 10, dans lequel (i) ledit polypeptide comprend en outre un résidu G précédant immédiatement ladite séquence d'acides aminés, dans un mode de réalisation comprend en outre la séquence d'acides aminés MG précédant immédiatement ladite séquence d'acides aminés ; (ii) ledit polypeptide comprend en outre un peptide de transglutaminase, dans un mode de réalisation comprenant la séquence d'acides aminés YRYRQ (SEQ ID NO : 15) ; (iii) ledit polypeptide comprend en outre un peptide étiquette, dans un mode de réalisation comprenant la séquence d'acides aminés (His)6 (SEQ ID NO : 5), dans un mode de réalisation supplémentaire (His)8 (SEQ ID NO : 6) ; et/ou (iv) ledit polypeptide comprend en outre au moins un peptide de liaison.

12. Polypeptide de fusion comprenant un polypeptide selon l'une quelconque des revendications 9 à 11 et un agent de liaison.

13. Complexe polypeptidique comprenant un premier partenaire d'une paire d'affinité (premier partenaire d'affinité) ; et un second partenaire de la paire d'affinité (second partenaire d'affinité) dans lequel (i) ledit premier partenaire d'affinité est un polypeptide selon la revendication 9 et ledit second partenaire d'affinité est un polypeptide comprenant la séquence d'acides aminés de SEQ ID NO : 2 ou une séquence au moins identique à 85 % à celle-ci ; ou (ii) ledit premier partenaire d'affinité est un polypeptide comprenant la séquence d'acides aminés de SEQ ID NO : 1 ou une séquence au moins identique à 85 % à celle-ci et ledit second partenaire d'affinité est un polypeptide selon la revendication 10.

14. Polynucléotide codant pour un polypeptide selon l'une quelconque des revendications 9 à 11, et/ou un polypeptide de fusion selon la revendication 12.

15. Kit comprenant
(I) un polypeptide selon la revendication 9 (premier partenaire d'affinité) ou un polynucléotide codant pour celui-ci ; et/ou
(II) un polypeptide selon la revendication 10 (second partenaire d'affinité) ou un polynucléotide codant pour celui-ci ;
compris dans un boîtier.
